# EUROPEAN PATENT SPECIFICATION

(11) **EP 2 661 212 B1**
(45) Date of publication and mention of the grant of the patent: **20.12.2017**
(21) Application number: 12817804.3
(22) Date of filing: 19.07.2012
(51) Int. Cl.: A61B 3/00, B29D 11/00, B29C 31/00

(54) **METHODS AND SYSTEMS FOR MANUFACTURING CONTACT LENSES**
VERFAHREN UND SYSTEME ZUR HERSTELLUNG VON KONTAKTLINSEN
PROCÉDÉS ET SYSTÈMES DE FABRICATION DE LENTILLES DE CONTACT

(30) Priority: 28.07.2011 US 201161512797 P
(43) Date of publication of application: 13.11.2013
(73) Proprietor: CooperVision International Holding Company, LP, St. Michael (BB)
(72) Inventor: NIU, Xuxian, Pleasanton, CA 94588 (US); RADWANSKI, Ryszard, Pleasanton, CA 94588 (US); SARJA, Henry, Pleasanton, CA 94588 (US); BAKER, Eddie, E., Atwater, CA 95301 (US); HUFMAN, James, R., Columbia, CA 95310 (US); WENZEL, Roger, R., Jarrell, TX 76537 (US); MEIGS, Theodore, V., Twain Harte, CA 95383 (US)
(74) Representative: Abel & Imray
(86) International application number: PCT/US2012/047357
(87) International publication number: WO 2013/016124

(56) References cited:
- WO-A1-2008/149404
- GB-A- 2 463 901
- US-A- 5 476 111
- US-A- 5 658 602
- US-A- 5 744 357
- US-A- 5 836 323
- US-A- 6 007 229
- US-A- 6 071 112
- US-A1- 2003 234 456
- US-A1- 2005 062 179
- US-A1- 2007 243 277
- US-B1- 6 558 584

## Description

### Field of the Invention

The invention relates to manufacturing contact lenses. More specifically, the invention relates to new methods and systems for manufacturing contact lenses.

### Background of the Invention

Contact lenses are often fabricated using mold assemblies in which a male mold section and a female mold section come together to form a lens-shaped cavity between the mold sections. US-A-5658602 discloses a known prior art.

Typically, a mold assembly for producing a single contact lens includes a female mold section having a concave optical surface defining an anterior surface of a lens to be made, and a male mold section having a convex optical surface defining a posterior surface of the lens to be made. When individual male and female mold sections are assembled together, a contact lens shaped cavity is formed between the concave surface of the female section and the convex surface of the male section.

A contact lens precursor composition, for example, a polymerizable composition including one or more monomers, is placed or deposited within the lens shaped cavity. For example, the polymerizable composition may be placed in contact with the concave surface of a female mold section and a male mold section is placed on the female mold section so that the convex surface of the male mold section contacts the polymerizable composition and maintains the polymerizable composition in the lens shaped cavity. The polymerizable composition may be polymerized in the mold assembly to form a polymeric lens body. The polymeric lens body is removed from the mold sections and is further treated and eventually packaged for consumer use as a contact lens.

The male and female mold sections used in the above-mentioned contact lens manufacturing process are themselves commonly formed through the use of molding processes, including injection molding and lathing. These mold sections may be formed from thermoplastic materials, for example, such as polystyrene, polypropylene, ethylene vinyl alcohol polymers, other vinyl alcohol copolymers, other thermoplastic polymeric materials, and the like.

GB 2463901 discloses a process for manufacturing a contact lens and an apparatus for performing the process, the process comprising: placing a picker comprising a plurality of picker heads (protrusions) into engagement with a first pallet (formation) carrying an array of male lens mold sections having a first optical surface; securing the male lens mold sections to the picker heads (protrusions); separating the picker and the male lens mold sections from the first pallet (formation); moving the separated picker and the male lens mold sections into engagement with a second pallet (retaining unit) carrying an array of female lens mold sections having a second optical surface; and placing the male and female lens mold sections in contact with each other. The picker is moved into a position of alignment such that the plurality of picker heads (protrusions) are substantially coaxial with openings that hold the array of female lens mold sections in an alignment member of the second pallet (retaining unit), the array of picker heads (protrusions) matching the array of openings in the alignment member. As such, the male and female lens mold sections within their respective arrays are brought into close proximate alignment.

In conventional contact lens manufacturing processes, the lack of precise alignment between the male and female molds forming the contact lens mold assembly has caused significant lens edge damage and disadvantageously high prism on edge thickness. In addition, a significant amount of the polymerizable composition, which may be quite expensive, is wasted from mold overflow and spilled onto other components if the mold sections are not properly aligned. This increases the costs of manufacturing contact lenses. Additionally, misalignment of the mold sections may cause the finished lenses to be scrapped. Even relatively small amounts of misalignment between the mold sections can result in some lens edge damage and high prism on edge thickness and cause discomfort to the lens wearer.

It would be advantageous to provide new methods and systems for manufacturing contact lenses which address one or more of these and other concerns. US 2007/0243277 discloses a first and second mold parts having first and second mold housings and first and second mold surfaces. Leader pins in the first mold housing and leader bushings in the second mold housing cooperatively orientate the first and second mold parts.

### SUMMARY OF THE INVENTION

New methods and systems for manufacturing contact lens have been discovered which provide alignment, for example, precise alignment, such as perfect or substantially perfect alignment, of the male mold section and the female mold section forming a contact lens mold assembly in which a contact lens can be formed in the lens-shaped cavity formed between the male mold section and the female mold section. Having the male mold section and female mold section so aligned in the mold assembly, for example, precisely aligned, can provide substantial improvements relative to using a mold assembly with misaligned male and female mold sections. Such improvements can include, without limitation, reduced contact lens manufacturing costs, reduced lens edge damage, a reduced incidence of unacceptable prism, and the manufacture of contact lenses which are more comfortable to wear. The present methods and systems are highly adaptable to manufacturing large numbers of contact lenses using male and female mold sections which are aligned, for example, precisely or substantially precisely aligned in the mold assembly. The present methods and system can be used to manufacture a large number of contact lenses, for example, at least about 100, about 500 or about 1000 or more lenses. In one example, when the present invention is used to manufacture a large number of contact lenses, the yield or percentage of acceptable contact lenses manufactured in accordance with the present invention can be higher than for identical contact lenses manufactured in identical mold assemblies without the male and female mold sections of the mold assembly being aligned in accordance with the present invention.

The present invention provides a method for manufacturing a contact lens, according to claim 1.
Thus, the present invention provides a method for manufacturing a contact lens which comprises placing a picking head assembly comprising a picking head and a plurality of spaced apart alignment pins into engagement with a first pallet carrying or holding a male lens mold section and having a plurality of first holes sized and positioned to receive the plurality of spaced apart alignment pins. The placing step is effective so that the picking head comes into alignment with the male lens mold section as the plurality of spaced apart alignment pins are received by the plurality of first holes and before the picking head contacts the male lens mold section. The male lens mold section is then secured to the picking head. The picking head assembly and the secured male lens mold section are separated from the first pallet, and the separated picking head assembly and secured male lens mold section are moved into engagement with a second pallet carrying or holding a female lens mold section and having a plurality of second holes sized and positioned to receive the plurality of spaced apart alignment pins. The moving is effective so that the male lens mold section comes into alignment with the female lens mold section as the plurality of spaced apart alignment pins are received in the plurality of second holes and before the male lens mold section contacts the female lens mold section. The aligned male and female lens mold sections may then be placed in contact with each other.

The picking head has a longitudinal axis and the picking head assembly may move substantially freely in a plane perpendicular to the longitudinal axis of the picking head. The picking head may come into alignment with the male lens mold section without the picking head physically touching the male lens mold section.

The movement of the picking head assembly substantially freely in a plane perpendicular to the longitudinal axis of the picking head facilitates at least one of the coming into alignment of the picking head with the male lens mold section and the coming into alignment of the male lens mold section and the female lens mold section.

The plurality of alignment pins on the picking head assembly is received into a plurality of first holes, e.g., alignment holes, in the first pallet to provide alignment between the picking head and the male lens mold section.

In one example, the male lens mold section includes a first optical surface, for example, a generally convex optical surface, and the female lens mold section includes a second optical surface, for example, a generally concave optical surface, and the moving step is effective so that the male lens mold section comes into alignment with the female lens mold section without the first optical surface contacting the second optical surface.

The present method involves the use of male (first) and female (second) pallets configured to carry or hold male and female molds, respectively.

The first or male pallet, the second or female pallet and the picking head assembly may be configured so the optical surface, for example, the optical convex surface, of the male lens mold section does not come into contact with any surface, for example, with any hard surface that may damage the optical surface of the male lens mold, when the first pallet is carrying or holding the male lens mold section, or when the male lens mold section is secured to the picking head, or during the separating of the male lens mold section from the first pallet, or during the moving the separated male lens mold section into engagement with the second pallet carrying or holding the female lens mold section, or during the closing of the aligned male and female lens mold sections together.

In one example, the first pallet carries or holds a plurality of male lens mold sections, and/or the second pallet holds or carries a plurality of female lens mold sections.

In one example, the male mold sections may be formed by direct injection of material into individual cavities, such that each mold section can be attached to its own separate runner system.

In one example, a plurality of male molds are arranged to form a male mold flower, for example, comprising the male mold sections, elongated runners or gates (spokes) and a central hub. The mold sections and runners may be located in substantially a single plane, for example forming a substantially planar array of male mold sections and runners. The arrangement of the mold sections in the flower can be "balanced", meaning the length of the runners from the central hub to a mold section is about the same for all of the mold sections in the flower. Alternatively, the arrangement of the mold sections in the flower can be "unbalanced", meaning the length of the runners from the central hub to a mold section is not about the same for all the mold sections in the flower. A stem or sprue may be provided perpendicular to the substantially single plane in which the mold sections and runners of the flower are located.

A female mold flower comprising a plurality of female mold sections, elongated runners, and a central hub arranged similarly to that described herein with regard to the male mold flower may be provided.

The pallets include alignment features to prevent optical surface damage and to guide the mold flowers into position. Alignment features may comprise structural segments (for example, raised segments) that form a defined pathway for placement of the elongated runners to facilitate locating of the mold flowers onto the pallets. Both male and female pallets comprise alignment holes into which a plurality of elongated, spaced apart alignment pins of the picking head assembly may be inserted.

The first pallet may include at least one first structural feature to facilitate the picking head coming into alignment with the male lens mold section. The second pallet includes at least one second structural feature to facilitate the male lens mold section, which may be carried by the picking head, coming into alignment with the female lens mold section.

The first pallet may have a cavity sized and positioned to receive the male lens mold section. The first pallet may have a rim, for example, a circumferential rim, recessed within and partially defining the cavity, the rim being adjacent, or substantially adjacent, the plurality of first holes sized and positioned to receive the plurality of spaced apart alignment pins, and being effective to assist in alignment of the male lens mold section in the cavity of the first pallet without contacting the optical surface of the male lens mold section.

In one example, the male lens mold section is attached to a first elongated runner and the first pallet includes a plurality of spaced apart first raised segments forming a defined pathway for placement of the first elongated runner to facilitate locating the male lens mold section on the first pallet, for example, as desired.

The male lens mold section may be one of a plurality of male lens mold sections, each male lens mold section being attached to a different first elongated runner and the first elongated runners being secured to a central hub, and the first pallet including a plurality of spaced apart first raised segments forming a plurality of defined pathways for placement of each of the elongated runners in a different defined pathway to facilitate locating the male lens mold sections on the first pallet, for example, as desired.

The first pallet may comprise one or more openings to allow the elongated runner or runners to pass through the first pallet when the elongated runner or runners is/are detached from the male lens mold section(s).

In one example, the method comprises detaching the first elongated runner from the male lens mold section prior to the step of placing the picking head assembly into engagement with the first pallet carrying or holding the male lens mold section. In one example, the method comprises detaching a plurality of elongated runners from a plurality of male lens mold sections prior to the step of placing the picking head assembly into engagement with the first pallet carrying or holding a plurality of male lens mold sections.

The second or female pallet has a cavity sized and positioned to receive at least a portion of the female lens mold section. The second pallet has a circumferential raised portion, within the cavity sized and positioned to be effective in supporting the female mold section on the second pallet, and being effective in assisting in aligning the female lens mold section with the male mold section.

The female lens mold section may be attached to a second elongated runner and the second pallet may include a plurality of spaced apart second raised segments forming a defined pathway for placement of the second elongated runner to facilitate locating the female lens mold section on the second pallet, for example, as desired.

In one example, the female lens mold section is one of a plurality of female lens mold sections, each female lens mold section being attached to a different second elongated runner and the second elongated runners being secured to a second central hub, and the second pallet including a plurality of spaced apart second raised segments forming a plurality of defined pathways for placement of each of the second elongated runners in a different defined pathway to facilitate locating the female lens mold sections on the second pallet.

In one example, the female lens mold section includes a mold outside skirt, and the moving step is effective to contact the plurality of spaced apart alignment pins with the mold outside skirt prior to the plurality of spaced apart alignment pins being received by the plurality of second holes.

In one example, the securing step and/or the separating step comprises applying a vacuum or suction, for example, through a vacuum or suction system connected to the picking head assembly. After separating, the male lens mold section may be held to the picking head, for example, by vacuum or suction.

A polymerizable composition may be provided by a step comprising placing the polymerizable composition on the female lens mold section at one or more of the following times: before, during, or after the moving step.

After placing the aligned molds in contact with each other, a contact lens may be formed in a lens-shaped cavity between the male and female mold sections, i.e., in a mold assembly. The lens can be formed with the mold sections contacting each other only in their flanged regions and without the optical surfaces of the male and female mold sections touching or contacting each other.

A system for manufacturing a contact lens is also provided, according to claim 15.
Embodiments of the invention comprise a picking head assembly comprising a picking head and a plurality of spaced apart alignment pins; a first pallet carrying a plurality of male lens mold sections and having a plurality of first holes sized and positioned to receive the plurality of spaced apart alignment pins; a vacuum source operatively coupled to the picking head and being operable to secure the male lens mold section to the picking head; and a second pallet carrying a female lens mold section and having a plurality of second holes sized and positioned to receive the plurality of spaced apart alignment pins. The picking head assembly and secured male lens mold section are movable into engagement with the second pallet so that the male lens mold section comes into alignment with the
female lens mold section as the plurality of spaced apart alignment pins are received in the plurality of second holes.

The system may further comprise a polymerizable composition on the female lens mold section, for example, a polymerizable composition present between the male lens mold section and the female lens mold section.

The present systems may be structured or adapted to be useful in carrying out the methods of manufacturing a contact lens, for example, as described herein. Much of the disclosure set forth herein applies to both such systems and methods.

In one example, the first pallet includes at least one first structural feature to facilitate the picking head coming into alignment with the male lens mold section. In one example, the second pallet includes at least one second structural feature to facilitate the male lens mold section, which may be carried by the picking head through vacuum suction, coming into alignment with the female lens mold section.

In one example, the picking head assembly and the first pallet are sized and structured so that the picking head comes into alignment with the male lens mold section carried by the first pallet without the picking head physically touching the male lens mold section.

In one example, the male lens mold section includes a first optical surface and the female lens mold section includes a second optical surface, and the system is sized and structured so that the male lens mold section secured to the picking head comes into alignment with the female lens mold section held by the second pallet without the first optical surface contacting the second optical surface.

In one example, the picking head has a longitudinal axis and the picking head assembly is substantially freely movable in a plane perpendicular to the longitudinal axis of the picking head.

The male pallet may comprise rims in cavities that receive the male molds.

The female pallet may comprise ridges in the cavities that receive and retain the female molds.

In one example, the female lens mold section may include a mold outside skirt. The female mold section may have a mold outside skirt that: (i) fits over a ridge in a cavity on a female pallet, for example, and is effective in assisting in aligning and/or stabilizing the female mold on the female pallet, and (ii) is effective in assisting in aligning the male mold section and the female mold section.

Before the male lens mold section is brought into contact with the female lens mold section on the female pallet, the plurality of spaced apart alignment pins of the picking head assembly carrying the male mold section are first received in holes in the female pallet. The holes in the female pallet are configured to receive the plurality of spaced apart pins of the picking head assembly.

In one example of the system, the male lens mold section and the female lens mold section may comprise a polymeric material, for example, a thermoplastic polymeric material, such as those materials commonly or conventionally used in contact lens mold sections. The thermoplastic polymeric material of the mold sections may comprise a polar thermoplastic polymeric material, such as, for example, an ethylene vinyl alcohol polymer or polybutylene terephthalate (PBT).

The first and second pallets may be made of any suitable material or combination of materials. In one example, the first and second pallets may comprise a polymeric material, for example, a thermoplastic polymeric material.

The mold sections and pallets may be made of compatible materials, for example, to facilitate the desired structure and functioning of the mold sections and pallets in accordance with the present invention. In one example, the male and female mold sections may comprise the same material. The first pallet and the second pallet may comprise the same material.

In one example, lens bodies of the contact lenses manufactured according to the present invention are cast molded lens bodies that include an anterior surface and a posterior surface. The lens bodies may or may not be surface modified or treated. One or more of the surfaces of the lens bodies may be treated with plasma or subjected to other surface treatment or modification. The surfaces of the lens bodies may be free of plasma treatment or other surface treatment or modification.

The contact lenses manufactured according to the present invention may include a lens body, for example, comprising a soft or water swellable, e.g., hydrogel polymeric material. In one example, the lens body comprises a polymeric silicone hydrogel material.

In one example, the polymeric material comprises units from a silicon-containing monomer, for example, from at least one silicon-containing monomer, such as from two silicon-containing monomers having different molecular weights, and possibly different chemical structures.

In one example, the present contact lenses comprise only one silicon-containing monomer having a relatively high molecular weight, such as, for example, greater than 3,000 daltons, or greater than 5,000 daltons, or greater than 10,000 daltons. This example, that is the example comprising one silicon-containing monomer, may be particularly useful for daily wear silicone hydrogel contact lenses that are typically discarded (rather than cleaned) on a daily basis.

The contact lenses manufactured in accordance with the present invention may be in any suitable configuration effective to satisfy the needs of the lens wearer. For example, the present lenses may have a single refractive power or two or more refractive powers, such as a bifocal or multifocal lens, or may have no refractive power. The present lenses can provide spherical corrections, aspherical corrections, cylinder corrections, wave front corrections, corrections of aberrations and the like. Without limitation, examples of useful cylinder correction lenses which may be formed in accordance with the present invention are disclosed in Back U.S. Patent No. 6,467,903. The present lenses can be configured to be rotationally stabilized, for example, including ballasts, other rotationally stabilizing features and the like.

The present lenses may be untinted, tinted, colored, for example, with iris-simulating patterns, and the like. The present lenses may have any suitable edge geometries, such as rounded edges, for example, fully rounded edges from posterior face to anterior face, rounded edges which include portions of the anterior face or the posterior face of the lens and the like. Such rounded edges or edge portions may be useful in enhancing the comfort and safety of wearing the present contact lenses, particularly during extended wear of such contact lenses. Without limitation, examples of useful contact lenses with rounded edges which may be formed in accordance with the present invention are disclosed in U.S. Patent No. 6,431,706.

Each and every feature described herein, and each and every combination of two or more of such features, is included within the scope of the present invention provided that the features included in such a combination are not mutually inconsistent as will be apparent from the context, this specification, and the knowledge of one of ordinary skill in the art. In addition, any feature or combination of features may be specifically excluded from any example of the present invention.

These and other aspects and advantages of the present invention will become apparent in the following detailed description, drawings, examples and claims, particularly when considered in conjunction with the accompanying drawings in which like parts bear like reference numerals.

### BRIEF DESCRIPTION OF THE DRAWINGS

FIG. 1 is a perspective view showing a male mold flower about to be loaded on a first or male pallet.
FIG. 1A is a perspective view of an alternate male mold flower.
FIG. 1B is a perspective view of another male mold flower.
FIG. 1C is a perspective view of a further male mold flower.
FIG. 2 is a perspective view showing the male mold flower positioned on the first pallet.
FIG. 3 is a perspective view showing the individual male lens mold sections positioned on the first pallet after being de-gated.
FIG. 4 is a perspective view showing a female mold flower about to be loaded on a second or female pallet.
FIG. 5 is a perspective view showing the female mold flower loaded on the female pallet.
FIG. 6 is a perspective view showing a picking head of a picking head assembly approaching the first pallet.
FIG. 6A is a cross-sectional view of a portion of a picking head assembly.
FIG. 6B is a perspective view of a picking head assembly (with a housing component shown transparent for illustration clarity).
FIG. 7 is a perspective view showing the picking head being aligned precisely with the male mold section positioned on the first pallet.
FIG. 8 is a perspective view showing the picking head of the picking head assembly fully engaged with a male lens mold section on the first pallet.
FIG. 9 is a cross-sectional view showing the picking head of the picking head assembly fully engaged with a male lens mold section on the first pallet.
FIG. 10 is a perspective view showing the picking head assembly with the picking head carrying the male lens mold section that has been picked up and separated from the first pallet.
FIG. 11 is a perspective view showing the picking head assembly, carrying the male mold section carried by the picking head, and moving toward the second or female pallet.
FIG. 12 is a perspective view showing the picking head assembly carrying the male mold section approaching the female pallet to align the male mold section and the female mold section.
FIG. 13 is a cross-sectional view of the picking head assembly carrying the male mold section moving into engagement with the second or female pallet carrying the female mold section.
FIG. 14 is a perspective view showing the picking head assembly, carrying the male mold section, engaged with the female mold and alignment of the male mold section and the female mold section is completed without the optical surfaces of the male and female mold sections physically contacting each other.
FIG. 15 is a cross-sectional view of the picking head assembly moved away from the female mold section to allow for dosing of the polymerizable composition onto the female mold section.
FIG. 16 is a cross-sectional view of the picking head assembly and the male and female mold sections after dosing of the polymerizable composition.
FIG. 17 is a somewhat schematic illustration showing (A) a symmetrical flower of runners and mold sections with all opposite angles equal; and (B) an asymmetrical flower of runners and mold segments with at least 2 opposite angles unequal. Although labeled with the male reference numerals, these arrangements of equal (A) and unequal (B) angles could represent either the male or the female lens mold sections and associated runners.

### DETAILED DESCRIPTION OF THE INVENTION

### Definitions

In the context of the present description, drawings, and additional disclosure claims, the following terminology will be used in accordance with the definitions described below. Unless expressly stated to the contrary herein, a number of terms set forth herein have the same or substantially the same definition as the same or substantially the same term defined in U.S. Patent Application Serial No. 12/894,941, published as publication number US2011/0085128, filed 9/30/10, entitled "SILICONE HYDROGEL CONTACT LENSES AND METHODS OF MAKING SILICONE HYDROGEL CONTACT LENSES", the disclosure of which in its entirety is incorporated herein by reference.

As used herein, the term "hydrogel" refers to a polymeric material, typically a network or matrix of polymer chains, capable of swelling in water or becoming swollen with water. A hydrogel can also be understood to be a material that retains water in an equilibrium state. The network or matrix may or may not be cross-linked. Hydrogels refer to polymeric materials, including contact lenses that are water swellable or are water swelled. Thus, a hydrogel may be (i) unhydrated and water swellable, or (ii) partially hydrated and swollen with water, or (iii) fully hydrated and swollen with water. The hydrogel may be a silicone hydrogel, a silicone-free hydrogel, or an essentially silicone-free hydrogel.

A "hydrophilic" substance is one that is water-loving or has an affinity for water. Hydrophilic compounds have an affinity to water and are usually charged or have polar moieties or groups that attract water.

As used herein, the terms "male mold" and "female mold" are abbreviated versions of and interchangeable with the terms "male lens mold section" and "female lens mold section", respectively.

A "monomer" refers to a single unit of a molecule containing one or more functional groups capable of polymerizing to combine with other molecules to form a polymer, the other molecules being of the same structure or different structures as the monomer. The monomer can be a relatively low molecular weight compound, for example a compound with a number average molecular weight less than 700 daltons. A monomer can also be a medium to high molecular weight compound, for example, a compound with a number average molecular weight of from about 700 daltons to about 2,000 daltons. A monomer can also be a high molecular weight compound, for example, a compound having a number average molecular weight greater than 2,000 daltons, such as, for example, greater than 5,000 daltons, or greater than 7,000 daltons.

A "polymer" refers to a material formed by polymerizing one or more monomers. As used herein, a polymer is understood to refer to a molecule that is not capable of being polymerized, but may be capable of being crosslinked to other polymers, for example, to other polymers present in a polymerizable composition or during the reaction of monomers to form other polymers in a polymerizable composition.

A "prepolymer" refers to a polymerizable or crosslinkable higher molecular weight compound. A prepolymer, as used herein can contain one or more functional groups. In one example, a prepolymer can be a series of monomers bonded together such that the overall molecule remains polymerizable or crosslinkable. For example, a prepolymer can be a compound with an average molecular weight greater than about 2,000 Daltons.

A "silicon-containing" component is a component that contains at least one silicon (Si) atom, for example, in a monomer or polymer. In addition to the silicon-containing compounds described herein, examples of still further silicon-containing components that may be useful in the present lenses can be found in U.S. Patent Nos. 3,808,178, 4,120,570, 4,136,250, 4,139,513, 4,153,641, 4,740,533, 5,034,461, 5,496,871, 5,959,117, 5,998,498, and 5,981,675, and U.S. Patent Application Publication Nos. 20070066706 A1, 20070296914 A1, and 20080048350 A1. The silicon-containing component can be a silicon-containing monomer a silicon-containing polymer.

A "siloxane-containing" component is a component that contains at least one unit of R₂SiO, where each R is independently either a hydrogen atom or a hydrocarbon group. Siloxane-containing components or siloxanes can have branched or linear backbones consisting of alternating silicon and oxygen atoms, i.e., -Si-O-Si-O, with side chains attached to the silicon atoms. The siloxane-containing component can be a monomer or polymer.

The term "silicone hydrogel" or "silicone hydrogel material" refers to a particular polymeric hydrogel that includes a silicon-containing component or a siloxane-containing component. For example, a silicone hydrogel is typically prepared by combining a silicon-containing material with conventional hydrophilic hydrogel precursors. A silicone hydrogel contact lens is a contact lens, including a vision correcting contact lens, which comprises a silicone hydrogel material.

### System and Method

The method and system of the present invention are defined by independent claims 1 and 15.

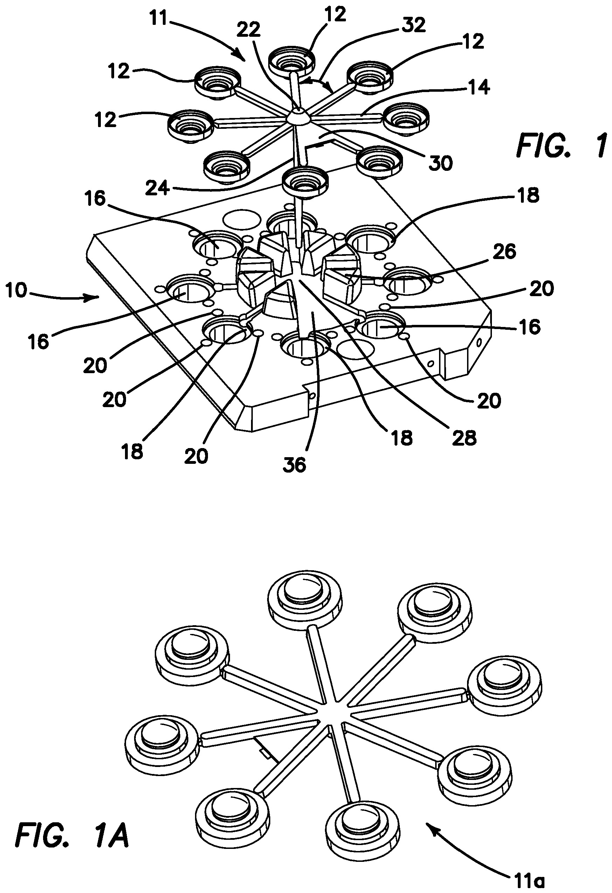

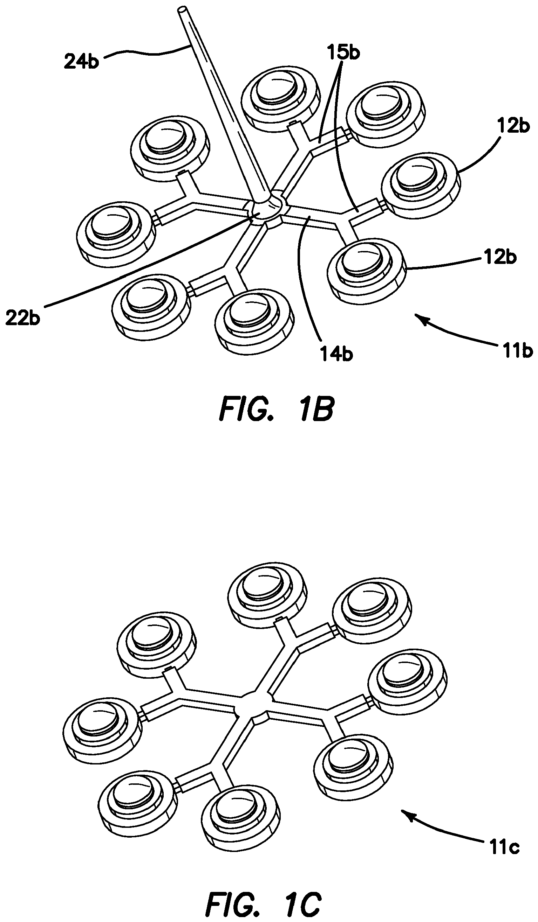

Referring now to FIG. 1, the first pallet **10** has cavities or depressions **16** therein structured or configured to receive male mold sections **12.** As illustrated in FIG. 1, each of the cavities **16** can be partially defined by an inwardly extending circumferential rim **18** of the first pallet **10.** The rims **18** can be sized and positioned to support the male mold sections **12** in the cavities **16.** In one example, the rims **18** can be sized and positioned to support the male mold sections **12** in the cavities 16 such that an optical surface (i.e., a surface of the male mold section used to form the posterior section of a contact lens) does not contact the first pallet **10** when the male mold section **12** is being held or carried by the pallet **10.** In one example, at least one cavity 16 or all of the cavities 16 may extend through the first pallet 10.

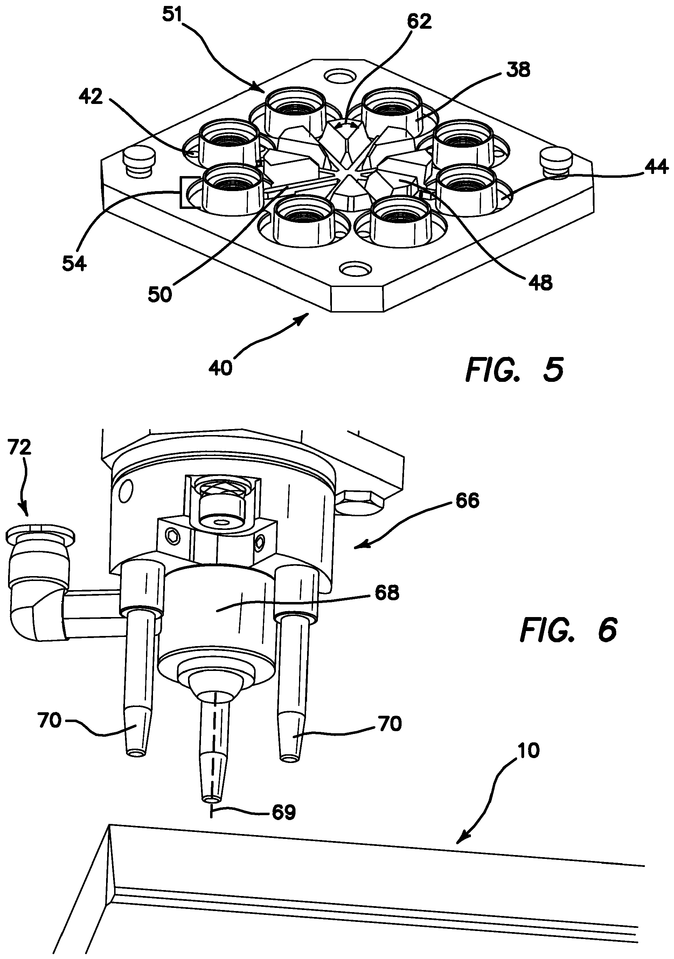

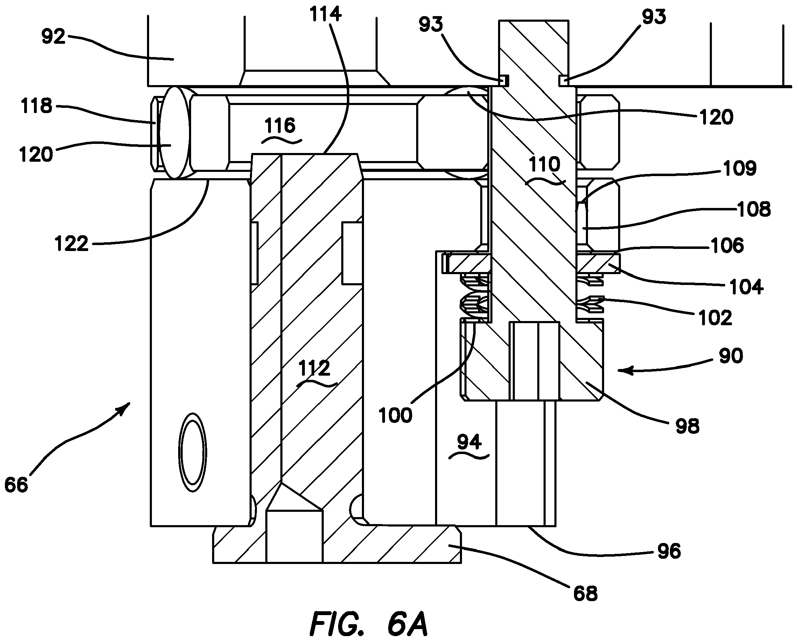

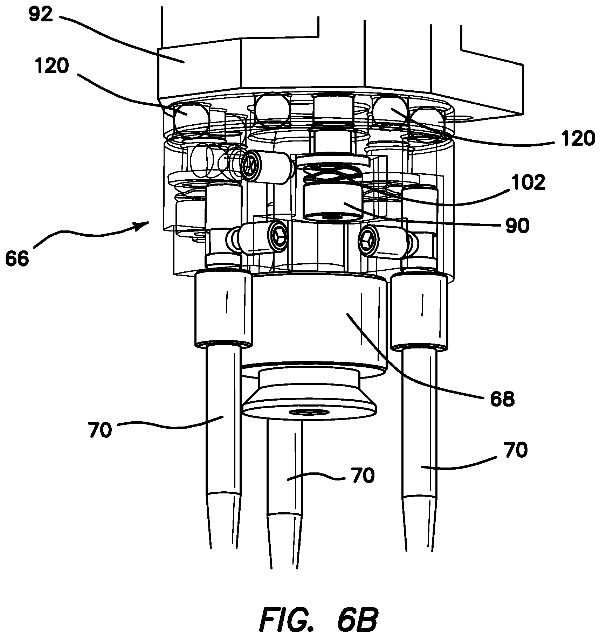

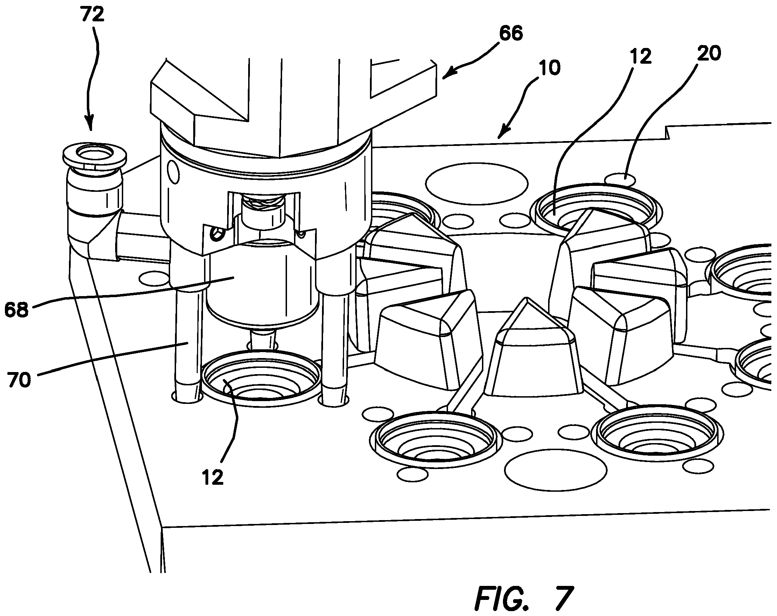

As illustrated in FIG. 1, each rim **18,** partially defining each of the cavities **16,** can be located substantially adjacent a plurality of spaced apart first alignment holes **20** in the first pallet **10** sized and positioned to receive the plurality of spaced apart alignment pins **70,** as is described hereinafter (see discussion with regard to FIG. 6 and FIG. 7).

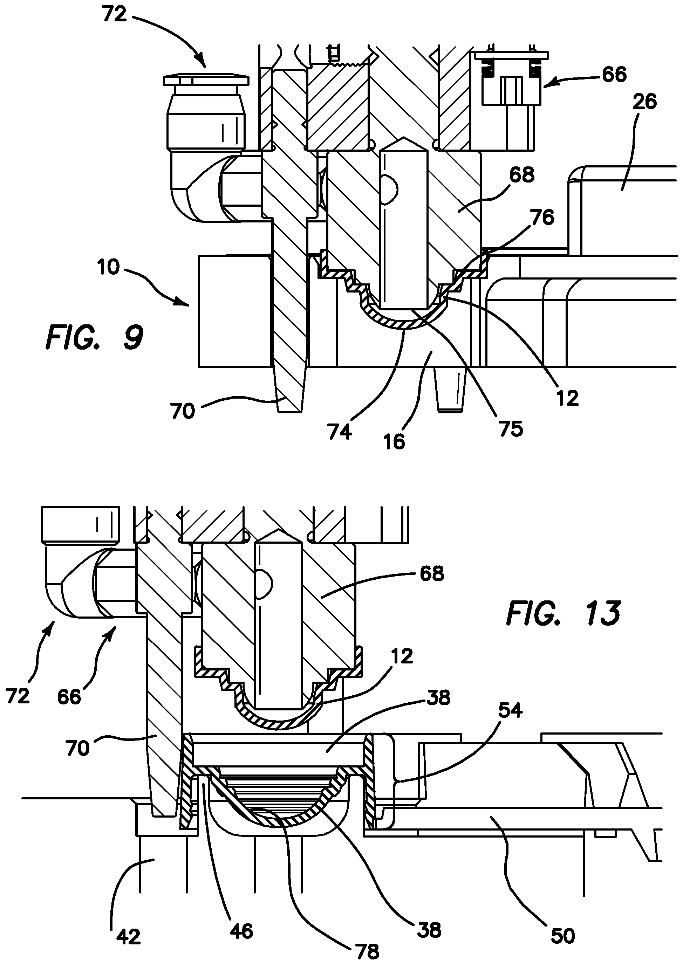

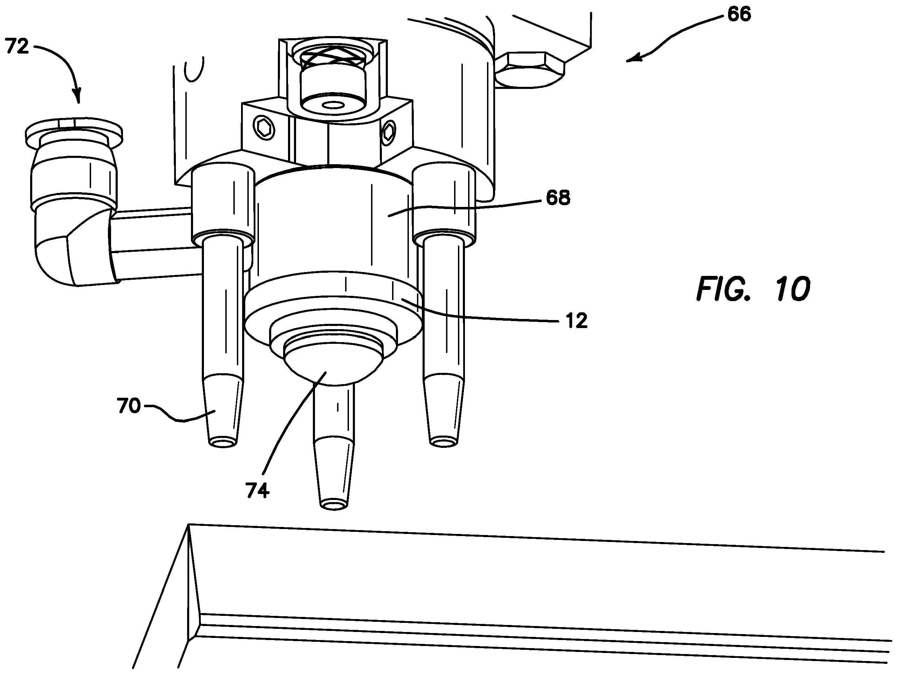

The first holes **20** are effective to assist or facilitate the alignment of the male mold section **12** in the cavity **16** without damaging the optical surface **74** (see FIG. 9 and FIG. 10) of the male mold **12** and without causing contact between the optical surface **74** of the male mold **12** and any other surface.

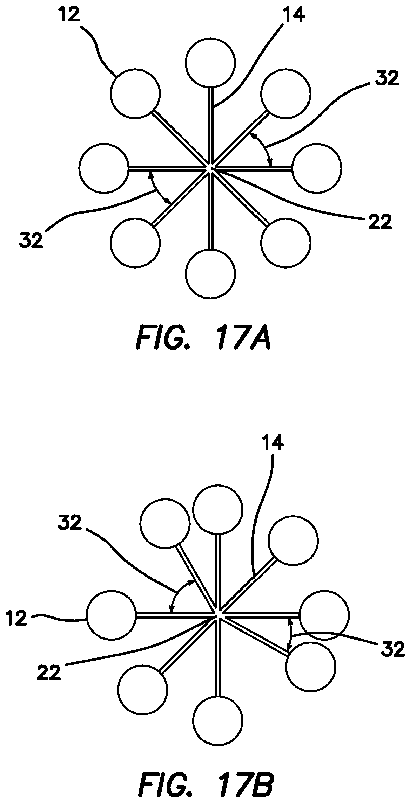

As shown in FIG. 1, above the first or male pallet **10** is a male mold flower **11,** comprising interconnected male mold sections **12,** about to be placed on the male pallet **10.** The male pallet **10** can be designed to accommodate a plurality of male lens mold sections **12** at the same time. The male mold sections **12** on male mold flower **11** can be interconnected to one another through a gate structure. As shown, according to one example, the gate structure may comprise a central hub **22** and elongated runners **14.** As illustrated in FIG. 1, each individual male lens mold section **12** can be connected to an elongated runner **14** and the runners **14** can join together at the central hub **22.** This configuration (hub **22** and elongated runners **14**) resembles a flower-like arrangement with the hub **22** as the bud and the runners **14** as the petals, the distal tips of the petals being the individual mold sections **12.** As shown in Fig. 1, a sprue or rod 24 may be provided as part of the male mold flower **11,** for example, attached or secured to hub 22, and located perpendicular to the plane in which the male mold sections **12** and elongated runners **14** are located. Sprue **24** may be useful in moving the male mold flower 11 in place on the male pallet **10.** Also as illustrated in FIG. 1, there can be an angle **32** between any two runners **14.** This angle **32** may be equal between any two adjacent runners for a symmetric arrangement or it may different. See FIG. 17 for a schematic representation of various examples, as discussed further herein.

The male mold flower **11** may have a different or alternate configuration relative to the male mold flower **11** shown in Fig. 1. A number of such different or alternate configurations are shown in Figs. 1A, 1B and 1C, it being understood that other different or alternate configurations for the mold flower are possible and may be included within the scope of the present invention.

For example, as shown in Fig. 1A, alternate male mold flower **11A** may be provided. Except as expressly described herein, male mold flower **11A** may be structured and may function substantially similarly to male mold flower **11.** The primary difference between male mold flower **11A** and male mold flower **11** is that male mold flower **11A** does not include a sprue, such as sprue **24** of male mold flower **11.**

Another configuration of the male mold flower is shown in Fig. 1B. Except as expressly described herein, male mold flower **11b,** shown in Fig. 1B, may be structured and may function substantially similarly to male mold flower **11.** The primary difference between male mold flower **11b** and male mold flow **11** is the configuration of the elongated runners **14b.** In particular, as shown in Fig. 1B, each of the elongated runners **14b** may be attached at one end to hub **22b.** A sprue **24b** may also be attached to hub **22b.** The other end of each of the runners **14b** may be formed into two branches **15.** Each of the branches **15** may be attached to a different male mold section **12b.**

In the configuration shown in Fig. 1B, the male mold flower **11b** includes 8 male mold sections **12b.** This is the same number of male mold sections as is included in male mold flower **11.** However, only 4 elongated runners **14b** are directly attached to hub **22b,** whereas 8 elongated runners **14** are directly attached to hub **22,** as shown in Fig. 1.

Fig. 1C illustrates a further male mold flower **11c.** Except as expressly described herein, male mold flower **11c** may be structured and may function similarly to male mold flower **11b**. The primary difference between male mold flower **11c** and male mold flower **11b** is that male mold flower **11c** does not include a sprue, such as sprue **24b** of male mold flower **11b.**

As shown in FIG. 1, each male lens mold section **12** can be attached to a different first elongated runner **14,** and the first or male pallet **10** can include a plurality of spaced apart first raised segments **26** forming a plurality of defined pathways for placement of each of the first elongated runners **14** in a different defined pathway to facilitate locating the male lens mold sections **12** on the first pallet **10.** In this example, the raised segments **26** resemble raised tapered wedges. In another example, the first pallet **10** may comprise at least two raised segments **26** forming at least one defined pathway for placement of at least one first elongated runner **14** to facilitate locating at least one male mold section **12** on the first pallet **10.** In yet another example, the first pallet **10** may comprise two raised segments **26** forming a single defined pathway for placement of a single first elongated runner **14** to facilitate locating a single male mold section **12,** or to facilitate locating a plurality of male mold sections **12** on the first pallet **10.**

One or more openings **28** can be provided in the first pallet **10** to accommodate insertion of the male mold flower **11** therein. After the male lens mold sections **12** have been placed in the male pallet **10,** the gate structure comprising the runners **14** and hub **22** may be disconnected from the male mold sections **12.** In one example, the runners **14** and hub **22** may be removed through opening **28.** For example, the runners **14** and hub **22** may be removed through opening **28** by allowing the runners **14** and hub **22** to drop through the opening **28,** or by mechanically pushing the runners **14** and hub **22** through opening **28,** or by using vacuum or suction to pull the runners **14** and hub **22** through the opening **28.** Optionally, as illustrated in FIG. 1, in one portion **36** of the male pallet **10,** the opening **28** can be especially noticeable because one of the raised segments **26** may be missing. This portion **36** with the missing raised segment **26** on the male pallet **10** matches up with an area of webbing **30** between two elongated runners **14** such that the male mold flower **11** only fits on the male pallet **10** in one particular way. This may be important as it can allow matching up specific male lens mold sections **12** formed in specific cavities of the injection molding system with specific female lens mold sections formed in specific cavities of the injection molding system. For example, different male lens mold sections **12** can be placed in different cavities **16** of the first pallet **10** to ensure that the correct male lens mold section is later combined with the correct female lens mold section, in order to be used to make contact lenses having different features, e.g., different optical properties.

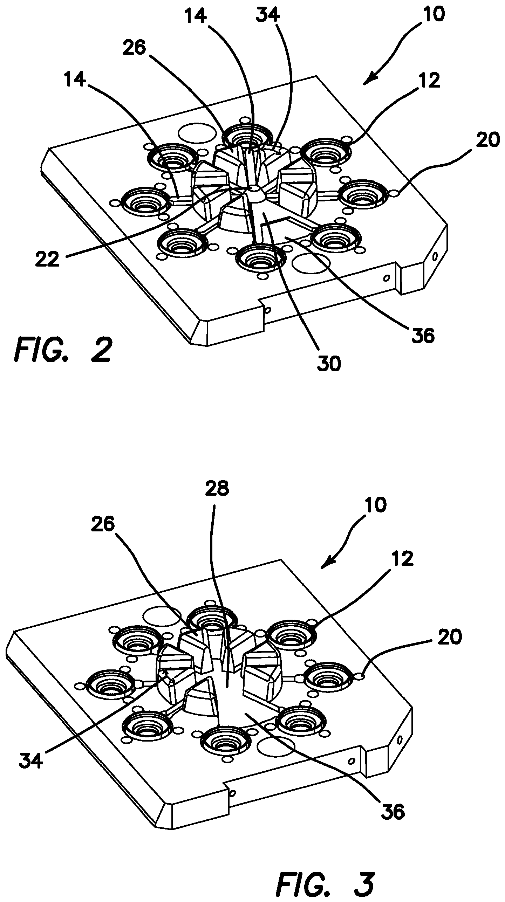

Referring now to FIG. 2, the male mold flower **11** comprising the gate structure **14, 22** interconnecting the various individual male lens mold sections **12** has been placed on, e.g., lowered onto, the male pallet **10.** As compared to FIG. 1, the elongated runners **14** interconnecting the various male lens mold sections **12** are now between the raised segments **26** on the male pallet **10** used to align and guide their placement. The webbed section **30** between two of the runners **14** fits above the missing wedge portion **36.** An angle **34** formed by the raised segments **26** (triangular wedges according to one example, as shown) substantially corresponds with an angle **32** formed between the elongated runners **14** (as shown in FIG. 1) in a manner such that each raised segment **26** fits between two elongated runners **14.**

Referring now to FIG. 3, the gate structure **14, 22** interconnecting the various male lens mold sections **12** has been removed. Removal of the gate structure **14, 22** is optional. The male lens mold sections **12** are present on the male pallet **10** in their respective cavities **16** waiting to be picked up. Openings **28, 36** remain where the gate structure **14, 22** has been removed.

Optionally, the male pallet **10** may comprise multiple interconnected openings **28, 36** such that vacuum or suction can pull the gate structure **14, 22** through the openings **28, 36** substantially intact, without the runners **14** collapsing.

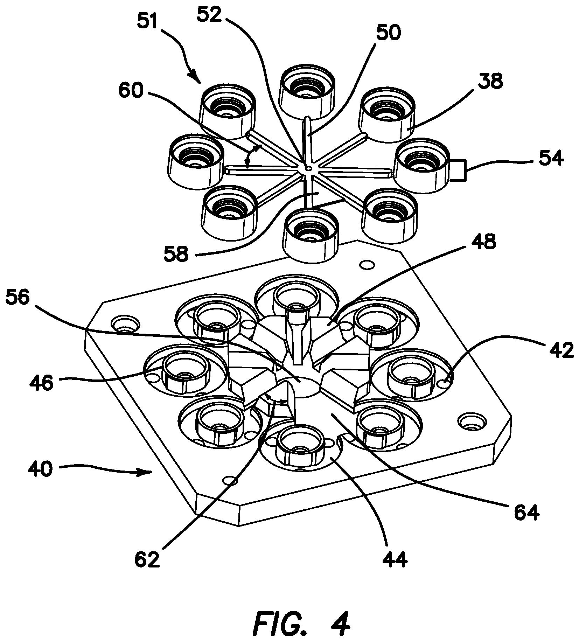

Referring now to FIG. 4, as shown in FIG. 4, the second or female pallet **40** can comprise a plurality of cavities **44,** each cavity **44** sized and configured to receive a female lens mold section **38.** The second pallet **40** comprises at least one cavity **44.** In another example, the second pallet 40 can comprise a single cavity **44.**

In the example illustrated in FIG. 4, a plurality of female lens mold sections **38** is provided. Each female lens mold section **38** can be attached to a different second elongated runner **50,** and the second pallet **40** can include a plurality of spaced apart second raised segments **48** forming a plurality of defined pathways for placement of each of the second elongated runners **50** in a different defined pathway to facilitate locating the female lens mold sections **38** on the second pallet **40.** As in the example illustrated in FIG. 4, each second elongated runner **50** of each female lens mold section **38** may be secured to a central hub **52.**

The runners **50** and hub **52** collectively form a gate structure. Other examples of the gate structure interconnecting the female lens mold sections to each other are also possible. Optionally, there can also be a sprue of the female mold flower similar to the sprue **24** of the male mold flower. It is not visible in the figures.

As shown in FIG. 4, female mold flower **51** can comprise a gate structure **50, 52** interconnecting a plurality of female lens mold sections **38.** According to one example, as shown, the second raised segments **48** can take the form of substantially triangular wedges. The angle **62** formed by the raised segments **48** corresponds with an angle **60** between two adjacent elongated runners **50** in a manner such that each raised segment **48** fits between two elongated runners **50.**

As with the first pallet **10,** in another example, the second pallet **40** may comprise at least two raised segments **48** forming at least one defined pathway for placement of at least one elongated runner **50** to facilitate locating at least one female mold section **38** on the second pallet **40.** In yet another example, the second pallet **40** may comprise two raised segments **48** forming a single defined pathway for placement of a single elongated runner **50** to facilitate locating a single female mold section **38,** or to facilitate locating a plurality of female mold sections **38** on the second pallet **40.**

As illustrated in FIG. 4, optionally, on one or more portions **64** of the female pallet **40** the raised segment **48** can be missing in order to accommodate webbing **58** between one or more sets of two adjacent elongated runners **50.**

The second or female pallet **40** can optionally further comprise one or more through openings **56** (a singular opening **56** is shown in FIG. 4) through which the gate structure **50, 52** optionally can be removed. For example, the gate structure **50, 52** may be removed through opening(s) **56** by allowing the runners **50** and hub **52** to drop through the opening(s) **56,** or by mechanically pushing the runners **50** and hub **52** through opening (s) **56,** or by using vacuum or suction to pull the runners **50** and hub **52** through the opening(s) **56.**

Each cavity **44** on the female pallet **40** can optionally comprise a circumferential raised portion, or ridge, **46** sized and positioned to receive one female lens mold section **38.** The circumferential raised portion **46** of each cavity **44** can be substantially adjacent a plurality of second alignment holes **42.** The plurality of second holes **42** are effective to assist or facilitate the alignment of a female mold section **38** with a male mold section **12** in a cavity **44,** as is discussed hereinafter.

As shown in FIG. 4, the raised portion or ridge **46** in the cavity **44** on the female pallet **40** can be inside the holes **42** of the pallet. The holes **42** are also shown within the cavity **44,** although examples where the holes **42** are outside the cavity **44** are also possible.

In one example (not shown) the raised portion or ridge **46** may be positioned outside the holes **42.** In this example, a plurality of elongated alignment pins of a picking head may pass through holes in an alternate example of a female mold section (not shown), placed over the ridge, before entering the pin holes in the female pallet.

The circumferential raised portion or ridge **46** is effective to assist in alignment of the female mold section **38** in the cavity **44.** In one example, each female lens mold section **38** can include an outside skirt **54** configured to fit over the circumferential raised portion or ridge **46.**

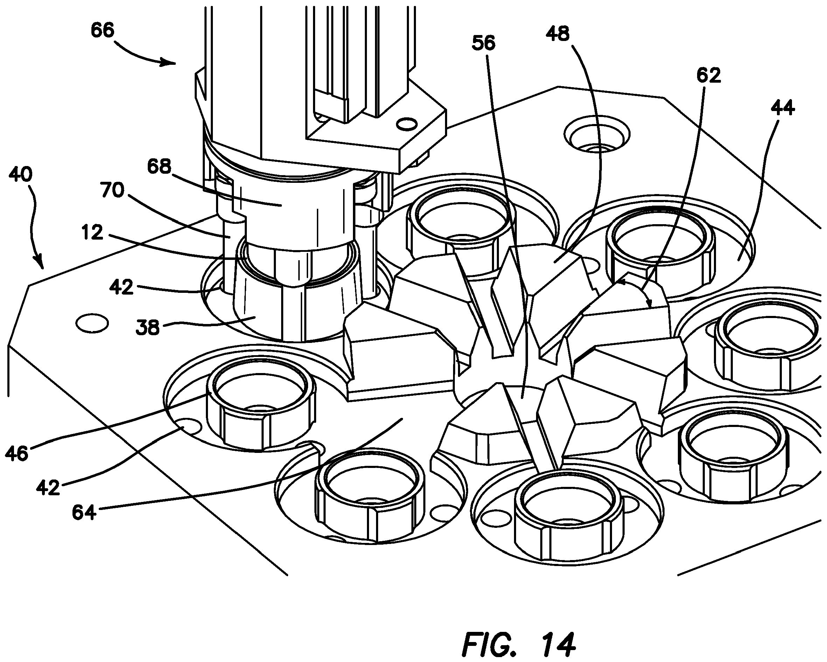

As shown in FIG. 4 and also FIG. 14, the female pallet **40** may comprise just one through opening **56** or central hole. Optionally, the gate structure **50, 52** can be detached from each of the female mold sections **38.** In one example, the entire gate structure **50, 52** optionally can be removed through this one opening **56** or central hole. For example, the runners **50** and hub **52** may be removed through opening **56** by allowing the runners **50** and hub **52** to drop through the opening **56,** or by mechanically pushing the runners **50** and hub **52** through opening **56,** or by using vacuum or suction to pull the runners **50** and hub **52** through the opening **56.** The gate structure **50, 52** can be configured to collapse inward on itself in order to fit through the opening **56.**

Referring now to FIG. 5, the female mold flower **51,** comprising a gate structure **50, 52** interconnecting the plurality of female lens mold sections **38,** has been placed on, e.g., lowered onto, the female pallet **40.** The elongated runners **50** are now positioned within the defined pathways created by the raised segments **48.**

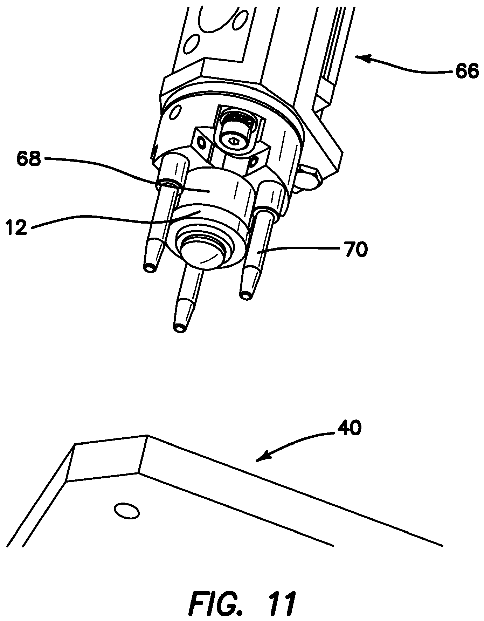

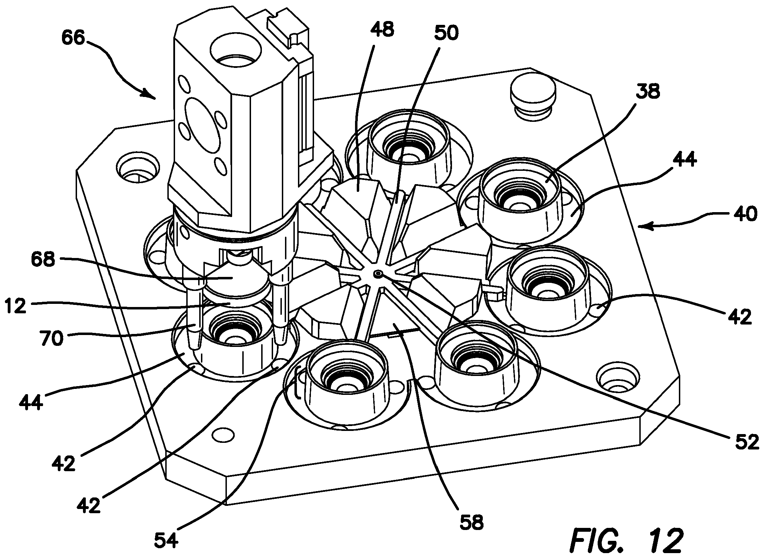

Referring now to FIG. 6, a picking head assembly **66** is provided in order to remove male lens mold sections **12** (see FIG. 7) from the first or male pallet **10,** transfer them, and place them in contact with the female lens mold sections **38** carried by a second or female pallet **40** (not shown in FIG. 6). This picking head assembly **66** comprises a picking head **68,** a plurality of alignment pins **70,** and a vacuum or suction system **72,** which can be of conventional design. The vacuum or suction system, shown schematically at **72,** provides sufficient force to remove a male lens mold section **12** (see FIG. 7) from the male pallet **10** and to hold the male lens mold section **12** to the picking head **68** during transport of the male mold section to the female pallet **40** (see FIG. 11) and during lowering the male lens mold section **12** (see FIG. 7) into contact with the female lens mold section **38** (see FIG. 12) in the contact lens manufacturing process. The picking head assembly **66** may move substantially freely in a plane perpendicular to the longitudinal axis or central longitudinal axis **69** of the picking head **68.** For example, if the picking head assembly **66** is lowered from directly over a horizontally situated male pallet **10,** the picking head assembly may move substantially freely in a horizontal plane or in a plane perpendicular to the direction in which the picking head **68** is lowered as the alignment pins **70** are received in the holes **20** (see FIG. 7) on the male pallet **10.**

Certain features of the picking head assembly **66** are shown in more detail in Figs. 6A and 6B.

As shown in Fig. 6A, a shoulder screw **90** may be a fixedly secured to top housing **92.** For example, the shoulder screw **90** can be fixedly secured to top housing **92** at one or more spaced apart locations **93,** as shown in Fig. 6A.

As shown in Figs. 6A and 6B, the shoulder screw **90** may extend downwardly into a hollow space **94** formed in connecting block **96.** The downward most part **98** of the shoulder screw **90** is enlarged and has an upper surface **100** which supports spring **102.** The other end of spring **102** is in contact with upper spring support **104,** which is positioned on shoulder screw **90.** A washer **106,** for example, made of a polymeric material, such as polytetraflouroethylene (Teflon), is deposed between upper spring support **104** and connecting block **96,** for example, as a friction retaining device.

Connecting block **96** includes a through opening **108** through which shoulder screw **90** passes. Through opening **108** is larger in diameter than the relatively thin portion **110** of the shoulder screw **90** within the through opening so that a gap or space **109** between the walls of through opening **108** and the thin portion **110** exists. This gap **109** between the through opening **108** and thin portion **110** of the shoulder screw **90** may be effective in facilitating, or even allowing, substantially free movement of the picking head assembly **66** in a plane perpendicular to the longitudinal axis of the picking head **68.**

As shown in Fig. 6A, connecting block **96** is also secured to picking head mount extension **112,** which is the upper portion of picking head **68.** The end **114** of extension **112** is located in a relatively large hollow space **116** defined by bearing housing **118.**

Bearing housing **118** contains a number of spaced apart ball bearings **120** which rotate between top housing **92** and the top surface **122** of connecting block **96.** The rotation of the ball bearings **120** may facilitate, or even allow, the picking head assembly **66** to move substantially freely in a plane perpendicular to the longitudinal axis of the picking head **68** as the alignment pins **70** are received in holes **20** of the male pallet **10.**

The spring **102** may create a preload on the connecting block **96,** may prevent undesired movement of the picking head assembly **66,** and may be effective in maintaining or stabilizing the position of the picking head assembly 68.

Fig. 6B shows many of the components of picking head assembly **66** described above with reference to Fig. 6A in a more complete illustration of the picking head assembly.

With reference to Fig. 6, the alignment pins **70** of the picking head assembly are designed to fit into first alignment holes **20** on the male pallet **10** and the second alignment holes **42** on the female pallet **40.** The pins **70** are placed into holes **20** on the male pallet **10** in order to align, for example, substantially precisely align, the picking head **68** with a male lens mold section **12** before removing the mold section **12** from the male pallet **10.**

The pins **70** are also placed into second alignment holes **42** on the female pallet **40** in order to align, for example, substantially precisely align the picking head **68** carrying a male lens mold section **12** with a female lens mold section **38** prior to lowering the male lens mold section **12** into contact with a female lens mold section **38** in the process of manufacturing a contact lens.

Referring now to FIG. 7, the picking head **68** of the picking head assembly **66** approaches the male mold section **12,** for example, by being lowered toward the male mold section. Then, as the picking head **68** is further moved toward the male mold section **12,** the pins **70** are received deeper into the holes **20.** At this point, with the pins **70** inserted into the pallet **10,** the picking head **68** is aligned, for example, precisely aligned, with the male mold section **12.** The picking head **68** can then be further lowered until it engages or contacts the male lens mold section **12.** Thus, the picking head **68** is aligned with the male mold section **12** before the picking head contacts the male mold section.

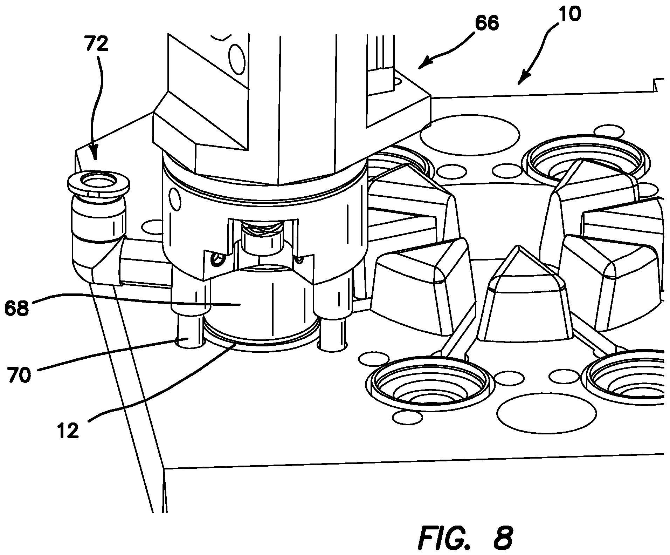

Referring now to FIG. 8, the picking head **68** has been moved, e.g., lowered, to engage the male lens mold section **12.** Optionally, at this point, vacuum or suction can be applied to the picking head **68** through the vacuum or suction port **72** in order to engage the male mold section **12.**

FIG. 9 is a cross-sectional view of the picking head **68** engaged with the male lens mold section **12.** With the picking head assembly **66** fully engaged with the male pallet **10** and the male lens mold section **12,** the optical surface **74** of the male lens mold section **12** is not in contact with any surface of the male pallet **10** or the pick head assembly **66,** as the male mold is contained within a cavity 16 of the male pallet **10.** The alignment pins **70** are fully inserted within the pin holes **20.** Optionally, as illustrated in the example illustrated in FIG. 9, the shape of the face **75** of the picking head **68** may substantially correspond to a substantial portion of the inner wall shape of the male lens mold section **12,** including flanged regions, such as inner flange region **76,** to facilitate the picking head **68** being able to remove the male lens mold section **12** from the male pallet **10** using vacuum or suction **72,** which is applied to the male mold section **12** through a hole in picking head **68.**

Referring now to FIG. 10, the picking head **68** portion of the picking head assembly **66,** as shown, has successfully picked up or removed the male lens mold section **12** from the male pallet **10,** has moved away from the male pallet **10** and is waiting to mate the male mold section with a corresponding female lens mold section **38** on a female pallet **40,** in the process of manufacturing a contact lens. In one example, the male lens mold section **12** can be mated with a female lens mold section **38** by the picking head **68** carrying the male mold section over to the female pallet **40.** In another example, the male lens mold section **12** can be mated with the female lens mold section **38** by the picking head **68** lifting and then lowering the male lens mold section as the female pallet **40** is substituted for the male pallet **10.** During the processes of either example, the male lens mold section **12** can be held to the picking head **68** through vacuum or suction **72.** Optionally, a polymerizable composition can be placed into the female mold member **38** prior to aligning the male mold member **12** and the female mold member **38.** Alternatively, the female mold member **38** and the male mold member **12** can be aligned, and then the male mold member **12** can subsequently be removed, the polymerizable composition can be added to the female mold member **38,** and the male mold member **12** can be re-aligned with the female mold member **38.**

Referring now to FIG. 11, the picking head **68** is shown moving the male lens mold section **12** to the female pallet **40.** The vertically elongated alignment pins **70** of the picking head assembly **66** extend distally further than the picking head **68** with attached male lens mold section **12.**

Referring now to FIG. 12, as the male lens mold section **12** carried by or held to the picking head **68** approaches a female lens mold section **38** on female pallet **40,** the alignment process starts when the two lens mold sections are still far apart. In one example, optionally, the alignment process can begin as the alignment pins **70** of the picking head assembly **66** come in contact with the outer surface of the outside skirt **54** of the female lens mold section **38.** However, it is not necessary for the pins **70** to contact the outside skirt **54** during the alignment process. In other words, alignment of the male lens mold section **12** and the female lens mold section **38** can be achieved by the alignment pins **70** entering the pin holes **42,** by the pins **70** contacting an outside portion of the female lens mold section **38,** or by both. The pins **70** can then move downward along the outer surface of the female lens mold section **38,** such as, for example, an outside skirt **54,** into the cavity **44** portion of the female pallet **40** and down into the holes **42** on the female pallet **40.**

Optionally, as shown in FIGS. 12 and 13, the plurality of pins **70** may be positioned in pin holes **42** inside the recessed cavity **44,** around the outside of the circumferential raised portion **46** over which the female lens mold section **38** with outer skirt **54** and optical surface **78** fits. As can be seen in FIG. 12, during the alignment process, no surface comes into contact with the optical surface **78** of the female lens mold section **38,** although the polymerizable composition may be in contact with the optical surface **78** of the female lens mold section **38** during the alignment process.

Referring further to FIG. 13, a cross-sectional view is provided of the pins **70** of the picking head assembly **66** lowered into the holes **42** in the female pallet **40.** The male lens mold section **12** is aligned, for example, precisely aligned, above the female lens mold section **38** prior to being put in contact with the female mold section. The picking head assembly **66** moves substantially freely in a horizontal plane or in a plane perpendicular to the longitudinal axis of the picking head 68 or in a plane perpendicular to the direction in which the picking head is lowered.

As illustrated in FIG. 12 and FIG. 13, the elongated runners **50** (or an alternative gate structure) may still be attached to the female mold section **38** and present on the second pallet **40** when the alignment process begins. Alternatively, in other examples (not shown) the runners **50** (or other gate structure) can be detached before the alignment process begins.

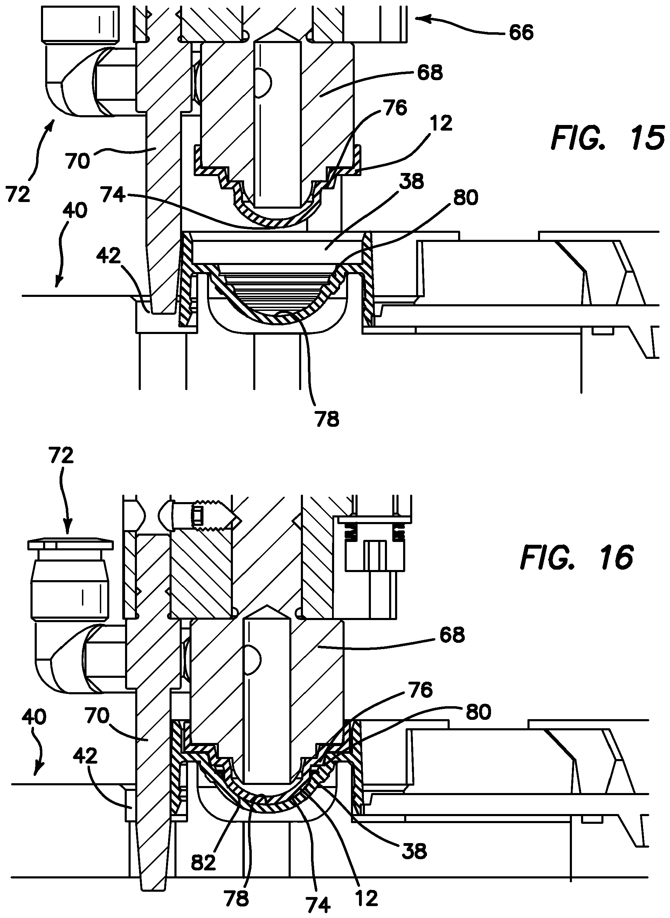

Referring now to FIG. 14, the system for manufacturing a contact lens is shown with alignment, for example, substantially precision alignment between the male mold section **12** and female mold section **38** completed. The pins **70** have been lowered completely into holes **42** on the female pallet **40** and the male lens mold section **12** carried by the picking head **68** has been lowered into contact with the female lens mold section **38** on the female pallet **40.** Even in this configuration, the optical surface **74** of the male lens mold section **12** does not contact the optical surface **78** of the female lens mold section **38,** although the polymerizable composition may be in contact with both the optical surface **74** of the male lens mold section **12** and the optical surface **78** of the female lens mold section **38** when the mold sections are in alignment. (See cross-sectional views in FIG. 15 and FIG. 16.)

Referring now to FIG. 15, a cross-sectional view is provided with alignment, for example, substantially precision alignment completed and the pins **70** of the picking head assembly **66** in the holes **42** on the female pallet **40.** The picking head **68** is shown above the female pallet **40,** as it is during the alignment process. Optionally, the picking head assembly **66** can place the male lens mold section **12** in contact with the female lens mold section **38,** for example, by placing in contact the flanged region **76** of the male mold section and the flanged region **80** of the female mold section. In one example, after placing the male mold section **12** and the female mold section **38** in alignment, the picking head assembly **66** can then pick up and move the male lens mold section **12** above the female lens mold section **38** to create a space between the male lens mold section **12** the picking head carries and the female lens mold section **38** on the female pallet **40,** for example to permit dosing of polymerizable composition into the female lens mold section **38.** After creating a space between the female lens mold section **38** and the male lens mold section **12,** the pick up head assembly **66** can then place the male mold section **12** and the female mold section 38 in alignment again.

Referring now to FIG. 16, a cross-sectional view is provided after the polymerizable composition dosing process is complete. The picking head **68** has been moved down, placing the male lens mold section **12** in contact with the female lens mold section **38.** The optical surface of the male lens mold section **74** does not contact the optical surface of the female lens mold section **78.** The polymerizable composition is located in the lens-shaped cavity **82** formed between the optical surface of the female mold section **78** and the optical surface of the male mold section **74** when the female mold section **38** and the male mold section **12** are placed in alignment, forming a mold assembly.

Optionally, once the male lens mold section **12** is in contact with the female lens mold section **38,** the picking head **68** or a separate device (not shown) can apply a downward force to the back to the male lens mold section **12** sufficient to engage an interference fit between the female lens mold section **38** and the lens mold section **12.** FIG. 16 shows a cross-sectional view after the picking head **68** has engaged the interference fit between the male lens mold section **12** and the female lens mold section **38.** Note that as compared to FIG. 15, the alignment pins **70** of the picking head assembly **66** have been lowered deeper into the holes **42** on the female pallet **40.** Contact between the male and female mold sections is only between the flanged region **76** of the male lens mold section **12** and the flanged region **80** of the female lens mold section **38,** which form the interference fit between the male and female mold sections.

In another example, optionally, the male lens mold section 12 can be affixed to the female lens mold section **38,** for example by applying a heat stake to melt a portion of each mold section and weld the mold sections together in a plurality of locations. The heat stake can be incorporated into the picking head assembly **66,** or can be a separate device. In yet another example, optionally an adhesive can be applied between the female lens mold section **38** and the male lens mold section **12** in order to affix the mold sections to each other.

Referring now to FIG. 17, schematic representations are shown of two of many possible arrangements of the mold flowers comprising a plurality of lens mold sections interconnected to each other through elongated runners joined to a central hub. Although reference numerals corresponding to the male lens mold sections and associated elements are provided, these schematic representations also represent possible arrangements for the female lens mold sections. In FIG. 17A, all angles **32** between any two adjacent elongated runners **14** are equal resulting in a symmetric arrangement. In FIG. 17B, all angles **32** between any two adjacent elongated runners **14** are not equal and an asymmetric arrangement is provided. However, an arrangement could also be provided in which some angles are equal and some angles are not equal.

De-gating refers to the process of detaching the runners **14, 50** from the molds **12, 38** and removing the runner and hub network (or other gate structure) such that the molds are alone and isolated from each other on the pallet. In one example, de-gating may involve detaching the runner and hub network **14, 22** or **50, 52** (or other gate structure) from the molds **12, 38** and then passing it through one or more openings **28** or **56** in the pallet **10, 40.** For example, the runner and hub network **14,** 22 or **50,52** may be removed through opening **28** or **56** by allowing the runner and hub network **14, 22** or **50, 52** to drop through the opening(s) **28** or **56** or by mechanically pushing the runner and hub network **14, 22** or **50, 52** through opening(s) **28** or **56,** or by using vacuum or suction to pull the runner and hub network **14, 22** or **50, 52** through the opening(s) **28** or **56.**

This de-gating process may be performed before or after the picking head **68** is aligned above the mold **12, 38** and before or after the picking head **68** is lowered to the mold, such as for picking up a male mold **12** or bringing the male mold into contact with the female mold **38.**

In one example, de-gating, in the form of detaching the first elongated runners **14** from the male lens mold sections **12,** may occur prior to the step of placing the picking head assembly **66** into engagement with the first pallet **10** carrying the male lens mold sections **12.**

In one example, de-gating, in the form of detaching the second elongated runners **50** from the female lens mold sections **38,** may occur prior to the step of moving the picking head assembly **66** (and male lens mold section **12** carried by it) into engagement with the female pallet **40.**

In one example, the first (male) pallet **10,** in addition to its cavities **16** with rims **18** (for fitting male mold) and pin holes **20** adjacent thereto, may also comprise a plurality of spaced apart first raised segments **26** forming a plurality of defined pathways for placement of each of the first elongated runners **14** in a different defined pathway to facilitate locating the male lens mold sections **12** on the first pallet **10.**

In one example, the first pallet **10** may comprise openings **28** to allow the elongated runners **14** to pass through the pallet **10** when the elongated runners **14** are detached from the male mold sections **12.**

Similarly, the second (female) pallet **40,** in addition to its cavities **44** with ridges **46** (for fitting female mold **30**) and pin holes **42** adjacent thereto, may also comprise a plurality of spaced apart second raised segments **48** forming a plurality of defined pathways for placement of each of the second elongated runners **50** in a different defined pathway to facilitate locating the female lens mold sections **38** on the second pallet **40.**

In one example, the second pallet **40** may comprise one or more openings **56** to allow the second (female) elongated runners **50** to pass through the pallet **40** when the elongated runners are detached from the female mold sections **38.**

The flower-like arrangement of the individual mold sections **12, 38** around a central hub **22, 52** may be either symmetric (as shown) or asymmetric. Accordingly, the system of cavities **16, 44** and plurality of spaced apart raised segments **26, 48** forming a plurality of defined pathways (for runners **14, 50**) on the pallets **10, 40** may be correspondingly symmetric (as shown in all drawings except FIG. 17B) or asymmetric (see FIG. 17B) such that the flower-like arrangement of mold sections **12, 38** and/or gate structure (hub **22, 52,** runners **14, 50**) matches up to fit on the pallet **10, 40** appropriately.

As shown in FIG. 17A, in a symmetric arrangement, the flower-like arrangement of mold sections **12** is such that each mold section **12** has another mold section **12** directly opposite it. In other words, all opposite angles **32** formed by the runners **14** interconnecting the mold sections are equal. To accommodate this, all angles **34** (see FIG. 2 and FIG. 3) formed by any two opposite wedge-shaped raised segments **26** on the pallet **10** (to assist in alignment of gate structure onto pallet) are equal.

However, there are still different variations of a symmetric arrangement. In one example, according to a first symmetric arrangement (as shown in all drawings except FIG. 17B), the presence of webbing **30, 58** between two of the runners **14, 50** makes it so that the flower can only be placed into the pallet **10, 40** in one position, as the pallet is made to accommodate the presence of the webbing **30, 58** by having only one missing wedge-shaped raised segment **36, 64.**

In another example, according to a second symmetric arrangement, the flower system could not have the webbing **30, 58** and the pallet **10, 40** could have all wedges **26, 48** (no missing wedge **36, 64**), such that it would be possible to rotate the flower and have the mold sections **12, 38** still fit within the pallet **10, 40,** providing several possible positions (distinguishable only to the extent that the properties of the individual molds or chemical compositions placed into the molds are different). That is, rotating the hub **22, 52** would cause different mold sections **12, 38** to fit into different mold cavities **16, 44** on the pallet **10, 40.**

In one example, in an asymmetric arrangement (as shown in FIG. 17B), the flower-like arrangement of mold sections **12** fits on the correspondingly asymmetric pallet in only one way, and rotating the hub **22** away from the singular aligned orientation would cause the mold sections **12** not to fit into the cavities **16** on the pallet.

In an asymmetrical flower, at least two of the opposite angles **32** (formed by the runners **14**) are different, and thus the shapes of the wedge-shaped raised segments **26** on the pallet **10** are different for at least two opposite segments, and no matter how the flower is rotated, the mold sections will only fit in the pallet **10** in one position.

By precisely controlling the vertical travel distance of the picking head assembly **66** that delivers the male mold **12** to the female mold **38,** perfect or near perfect alignment is achieved without the optical surfaces of the male and female mold sections coming into contact. Additionally, when a closing force is applied to the mold sections, the closing force can be closely monitored.

In one example, the step of closing the aligned male and female lens mold sections together may involve monitoring a force required to close the aligned male and female lens mold sections, determining whether or not the force required to close the male and female lens mold sections is within a specified range, and if the force required is outside the specified range, rejecting the aligned male and female lens mold sections and removing the mold sections from a manufacturing line.

Alignment can be performed before or after monomer dosing onto the female mold **38.** In one example, according to a first alternative, alignment is performed before monomer dosing. This involves aligning the molds, separating the molds for monomer dosing, and then placing the molds in contact again. In one example, according to a second alternative, alignment is performed after monomer dosing. This involves dosing monomer onto the female mold **38** (which may, but need not, be solitary or isolated from the male mold **12**) and then aligning the male and female molds and subsequently placing them in contact.

In the first alternative, alignment is immediately followed by dosing monomer. In the second alternative, alignment is immediately followed by placing the molds in contact.

In one example, after alignment is achieved, the male and female lens molds can be brought into contact, and their respective flanged regions **76, 80** can be engaged with one another. Closing the male mold section **12** and the female mold section **38** optionally can comprise applying force to the male mold section **12** to engage an interference fit between the male mold section **12** and the female mold section **38.** The closing force can be applied by the picking head **68** pressing down on the male mold section **12.** In this example, the solid picking head **68** head itself can physically press down on the male mold section **12** to engage the interference fit between the male mold section **12** and the female mold section **38.** The pressing force can be monitored, and it is possible to accept or reject a mold assembly based on the force required to engage the interference fit. In this example, the vacuum source **72** can be operatively coupled to the picking head **68** operable to secure the male lens mold section **12** to the picking head **68.**

Following closing to form an interference fit, the male mold section **12** may be placed into actual contact with the female mold section **38.** Even during this stage of actual contact between the male and female mold sections, contact of the optical surfaces **74, 78** of the molds may be avoided. Any contact that occurs between the molds **12, 38** may be at the edges or flanges **76, 80** of the molds while leaving their optical surfaces **74, 78** preserved and protected.

Subsequently, the male mold section **12** can be raised away from and out of contact with the female mold section **38.** Monomer may then be dosed onto the female lens mold **38.**

After the male lens mold section **12** has been placed in contact with, and affixed to the female lens mold section **38** forming a closed mold assembly, the closed mold assembly can be transferred to a tray using the vacuum **72** head. For example, using the vacuum or suction **72** to hold the male lens mold section **12** to the picking head **68** of the picking head assembly **66,** the picking head assembly **66** can be used to move the closed mold assembly to a tray.

In one example, when the male lens mold section **12** and the female lens mold section **38** of the closed mold assembly have both been de-gated from the runner and hub network of the male mold flower **14, 22** and the runner and hub network of the female mold flower **50, 52,** the closed mold assemblies can be transferred to a tray individually. In another example, when either the male lens mold section **12** and the female lens mold section **38** have not been de-gated from either the runner and hub network of the male mold flower **14, 22** or the runner and hub network of the female mold flower **50, 52,** i.e., when either or both the male mold section and the female mold section of a closed mold assembly remain attached to their respective runner and hub networks, all of the closed mold assemblies attached to a runner and hub network **14, 22** or **50, 52** can be transferred to a tray at the same time.

In one example, when the male and female lens mold sections are in contact, a form of energy may be applied to secure the male mold section **12** and the female mold section **38.** For example, the lens mold sections may be welded together by heat stake, laser, ultrasound, etc.

In one example, a form of energy may be applied to secure the male mold section **12** and the female mold section **38** before the vacuum **72** head (carrying or holding the male lens mold **12** to the picking assembly **66**) disengages from the male mold section **12.**

In one example, the energy may be applied before the plurality of spaced apart alignment pins **70** on the picking head assembly **66** are disengaged from the second (female) pallet **40.**

In one example, following the dosing of the polymerizable composition into the lens-shaped cavity and alignment of the female mold section **38** and the male mold section **12** to form the mold assembly, the mold assembly can be exposed to a form of energy, such as, for example, thermal radiation, UV light, and the like, in order to cause polymerization of the polymerizable composition, thus forming a polymeric lens body within the lens-shaped cavity of the mold assembly.

### Materials

In addition to what is explicitly recited herein, suitable materials and compositions for the polymerizable composition and the polymeric material may be found in the following publications and patents: U.S. Patent Application Publication No. 2011085128; U.S. Patent Application Publication No. 20060063852; U.S. Patent Application Publication No. 20070066706; U.S. Patent No. 7,320,587; and U.S. Patent No. 7,785,092.

In one example, either or both of the male and female lens mold sections may comprise one or more polymeric materials. Suitable polymeric materials for the mold sections include, but are not limited to, the following compositions and combinations thereof: thermoplastic materials, for example, such as polystyrene or polypropylene, and the like.

In one example, each of the male and female lens mold sections may comprise an injection molded thermoplastic polymeric material. Suitable thermoplastic materials include those in which the flow characteristics of the fluid thermoplastic polymeric material used to form the mold sections advantageously have an appropriate balance of high fluidity during introduction of the fluid thermoplastic polymeric material into a mold cavity (used to form the mold) and rapid cooling and/or solidification of the thermoplastic material once the cavity has been filled.

The thermoplastic material may be a thermoplastic polymeric material, for example, selected from any suitable such material or mixtures of such materials. For example, and without limitation, the thermoplastic polymeric material may comprise a polymer such as polyolefins, e.g., polypropylene, polyethylene, and the like, poly ethylene vinyl alcohol (EVOH), polyamides, poly oxy methylene, poly ethylene terephthalate, cyclic olefin co-polymers, polystyrene, polyvinyl chloride, copolymers of styrene with acrylonitrile and/or butadiene, acrylates, for example, poly methyl methacrylate, and the like, polyacrylonitrile, polycarbonate, polyesters, poly(4-methylpentene-1), and the like and mixtures thereof.

In one example, the polymeric material of the mold section can comprise a non-polar thermoplastic material, such as, for example, polystyrene or polypropylene. In another example, the polymeric material can comprise a polar thermoplastic material, such as, for example, polybutylene terephthalate, or an ethylene vinyl alcohol polymer, or a vinyl alcohol copolymer other than an ethylene vinyl alcohol polymer. In yet another example, the polymeric material can comprise a mixture of a polar thermoplastic material and a non-polar thermoplastic material.

In one example, the mold section may comprise a ethylene vinyl alcohol polymer (EVOH). For example, examples of the present mold sections, including the illustrated examples, can be made from an EVOH based resin publicly available under the tradename of SOARLITE.TM. S by Nippon Gohsei, Ltd. (Osaka, Japan). Various grades of EVOH with ethylene copolymerization ratio of about 25-50% by mole can be used in the present invention. Ethylene vinyl alcohol polymers are particularly useful, for example, from the viewpoint of ease of molding, providing a dimensionally stable mold and giving stable water wettability to the molded lens. "Soarlite" is an example of an ethylene-vinyl alcohol copolymer resin product.

In another example, the mold section may comprise a vinyl alcohol copolymer available under the tradename of G-POLYMER.TM. by Nippon Gohsei, Ltd. (Japan).

In yet another example, the mold section may comprise a form of polybutylene terephthalate (PBT).

Other suitable molding materials include polypropylene, polyethylene, polyamides, poly oxy methylene, poly ethylene terephthalates, cyclic olefin co-polymers, polystyrene, polyvinyl chloride, copolymers of styrene with acrylonitrile and/or butadiene, acrylates such as poly methyl methacrylate, polyacrylonitrile, polycarbonate, polyamides, polyesters, poly(4-methylpentene-1), and the like, and mixtures thereof.

Very useful mold materials are insoluble to a polymerizable composition and have contact angles to water at least at the part for forming one lens surface, not higher than about 90 degrees, by the sessile drop method. In one example, the contact angles may be about 65 degrees to about 80 degrees, by the sessile drop method. A contact lens formed using a mold material having surface contact angle smaller than 80 degrees shows particularly superior water wettability and stable performance in lipid deposition and the like. A mold material having surface contact angle smaller than 65 degrees is not advantageous because of difficulty in separating from the mold after polymerization, resulting in minute surface damage or fractures at an edge part of lens. A mold material soluble to monomer compositions is also difficult to use because of difficulty in separating the lens as well as rough lens surfaces and low transparency.

In the process of manufacturing a contact lens according to the present invention, a polymerizable composition may be placed on the female lens mold section. As the male lens mold section is moved into alignment with the female lens mold section and closes in upon it the polymerizable composition is positioned between the male lens mold section and the female lens mold section.

The polymerizable composition comprises at least one monomer. In one example, the polymerizable composition comprises at least one of a silicon-containing monomer.

While the present invention has been described with respect to various specific examples and embodiments, it is to be understood that the invention is not limited thereto and that it can be variously practiced within the scope of the following claims.

## Claims

1. A method for manufacturing a contact lens, the method being **characterised by** the combined steps of:
placing a picking head assembly (66) having a single picking head (68) and a plurality of spaced apart alignment pins (70) into engagement with a first pallet (10) carrying a plurality of male contact lens mold sections (12), each male lens mold section (12) having a first optical surface, the first pallet (10) having a plurality of first holes (20) sized and positioned to receive the plurality of spaced apart alignment pins (70), the placing being effective so that the picking head (68) comes into alignment with a single male lens mold section (12) carried by the first pallet (10) as the plurality of spaced apart alignment pins (70) are received by the plurality of first holes (20) and before the picking head (68) contacts the male lens mold section (12);
securing the single male lens mold section (12) to the picking head (68);
separating the picking head assembly (66) and the single male lens mold section (12) from the first pallet (10);
moving the separated picking head assembly (66) and the single male lens mold section (12) into engagement with a second pallet (40) carrying a female lens mold section (38) having a second optical surface (78), the second pallet (40) having a plurality of second holes (42) sized and positioned to receive the plurality of spaced apart alignment pins (70), the moving being effective so that the single male lens mold section (12) comes into alignment with the female lens mold section (38) carried by the second pallet (40) as the plurality of spaced apart alignment pins (70) are received in the plurality of second holes (42) and before the single male lens mold section (12) contacts the female lens mold section (38); and
placing the aligned male and female lens mold sections (12), (38) in contact with each other;
wherein the second pallet (40) also has a second cavity (44) sized and positioned to receive the female lens mold section (38), and a circumferential raised portion (46) within the cavity (44), the raised portion (46) being effective to assist in aligning the female lens mold section (38) in the cavity (44) with the single male lens mold section (12), without said single male lens mold section (12) contacting the second optical surface (78) of the female lens mold section (38).

2. The method of claim 1 which further comprises:
providing a polymerizable composition between the aligned male and female lens mold sections (12), (38), forming a mold assembly; and
reacting the polymerizable composition to form a polymeric contact lens body.

3. The method of claim 1, wherein the picking head (68) has a longitudinal axis and the picking head assembly (66) moves substantially freely in a plane perpendicular to the longitudinal axis of the picking head (68).

4. The method of claim 3, wherein the movement of the picking head assembly (66) substantially freely in a plane perpendicular to the longitudinal axis of the picking head (68) facilitates at least one of the coming into alignment of the picking head (68) with the single male lens mold section (12) and the coming into alignment of the single male lens mold section (12) and the female lens mold section (38).

5. The method of claim 1, wherein the first pallet (10), the second pallet (40) and the picking head assembly (66) are configured so that the first optical surface of the single male lens mold section (12) does not come into contact with any surface when the first pallet (10) is carrying the single male lens mold section (12), when the single male lens mold section (12) is secured to the picking head (68), during the separating step, during the moving step and during the closing step.

6. The method of claim 1, wherein the first pallet (10) has a first cavity (16) sized and positioned to receive the single male lens mold section (12), and a circumferential rim (18) recessed within the cavity (16), the rim (18) being substantially adjacent the plurality of first holes (20) sized and positioned to receive the plurality of spaced apart alignment pins (70), and the rim (18) being effective to assist in alignment of the single male lens mold section (12) in the cavity (16) without contacting the first optical surface of the single male lens mold section (12).

7. The method of claim 2, wherein the providing step comprises placing the polymerizable composition on the female lens mold section (38) at at least one of the following times: before the moving step, during the moving step, and after the moving step.

8. The method of claim 1, wherein the female lens mold section (38) includes a mold outside skirt (54), and the moving step is effective to contact the plurality of spaced apart alignment pins (70) with the mold outside skirt (54) prior to the plurality of spaced apart alignment pins (70) being received by the plurality of second holes (42).

9. The method of claim 1, wherein the first pallet (10) carries a plurality of male lens mold sections (12), and the second pallet (40) holds a plurality of female lens mold sections (38).

10. The method of claim 1, wherein the moving step is effective so that the single male lens mold section (12) comes into alignment with the female lens mold section (38) without the first optical surface contacting the second optical surface (78).

11. The method of claim 1, wherein the first pallet (10) includes at least one first structural feature to facilitate the picking head (68) coming into alignment with the single male lens mold section (12), and/or the second pallet (40) includes at least one second structural feature to facilitate the single male lens mold section (12) coming into alignment with the female lens mold section (38).

12. The method of claim 1, wherein the single male lens mold section (12) is attached to a first elongated runner (14) and the first pallet (10) includes a plurality of spaced apart first raised segments 26 forming a defined pathway for placement of the first elongated runner (14) to facilitate locating the single male lens mold section (12) on the first pallet (10).

13. The method of claim 12, wherein the first pallet (10) comprises an opening to allow the first elongated runner (14) to pass through the first pallet (10) when the elongated runner (14) is detached from the single male lens mold section (12).

14. The method of claim 1, wherein the plurality of spaced apart alignment pins (70) is positioned around the picking head (68).

15. A system for manufacturing a contact lens, the system comprising:
a picking head assembly (66) having a single picking head (68) and a plurality of spaced apart alignment pins (70), the picking head assembly (66) being structured to be secured to a single male lens mold section (12);
a first pallet (10) carrying a plurality of male contact lens mold sections (12) and having a plurality of first holes (20) sized and positioned to receive the plurality of spaced apart alignment pins (70);
a vacuum source (72) operatively coupled to the picking head (68) and being operable to secure a single male lens mold section (12) to the picking head (68); and
a second pallet (40) carrying a female lens mold section (38) and having a plurality of second holes (42) sized and positioned to receive the plurality of spaced apart alignment pins (70), the second pallet (40) having a second cavity (44) sized and positioned to receive the female lens mold section (38), **characterised by**
the picking head assembly (66) having a single picking head (68), and the second pallet (40) having a circumferential raised portion (46) within the second cavity (44), the raised portion (46) being effective to assist in aligning the female lens mold section (38) in the second cavity (44) with the single male lens mold section (12) secured to the picking head assembly (66) without the single male lens mold section (12) contacting a second optical surface (78) of the female lens mold section (38), wherein the picking head assembly (66) and secured single male lens mold section (12) are movable into engagement with the second pallet (40) so that the single male lens mold section (12) comes into alignment with the female lens mold section (38) as the plurality of spaced apart alignment pins (70) are received in the plurality of second holes (42).

16. The system of claim 15, wherein the picking head (68) has a longitudinal axis and the picking head assembly (66) is substantially freely movable in a plane perpendicular to the longitudinal axis of the picking head (68).

17. The system of claim 15, wherein the first pallet (10) has a first cavity (16) sized and positioned to receive the male lens mold section (12), and a circumferential rim (18) recessed within the cavity (16), the rim (18) being substantially adjacent the plurality of first holes (20) sized and positioned to receive the plurality of spaced apart alignment pins (70), and the rim (18) being effective to assist in alignment of the male lens mold section (12) in the cavity (16) without contacting the first optical surface of the male lens mold section (12).

18. The system of claim 15, wherein the plurality of spaced apart alignment pins (70) is positioned around the picking head (68).

## Patentansprüche

1. Verfahren zum Herstellen einer Kontaktlinse, wobei das Verfahren **dadurch gekennzeichnet ist, dass** es die folgenden kombinierten Schritte umfasst:
das Anordnen einer Anordnung mit Greifkopf (66), die einen einzelnen Greifkopf (68) und eine Vielzahl von beabstandeten Ausrichtungsstiften (70) aufweist, in Eingriff mit einer ersten Stapelplatte (10), die eine Vielzahl von männlichen Kontaktlinsenformabschnitten (12) trägt, wobei jeder männliche Linsenformabschnitt (12) eine erste optische Oberfläche aufweist, und wobei die erste Stapelplatte (10) eine Vielzahl von ersten Löchern (20) aufweist, die so bemessen und positioniert sind, um die Vielzahl von beabstandeten Ausrichtungsstiften (70) aufzunehmen, wobei das Anordnen bewirkt, dass der Greifkopf (68) in Ausrichtung mit einem einzelnen männlichen Linsenformabschnitt (12), welcher von der ersten Stapelplatte (10) getragen wird, kommt, wenn die Vielzahl der beabstandeten Ausrichtungsstifte (70) von der Vielzahl der ersten Löcher (20) aufgenommen werden und bevor der Greifkopf (68) den männlichen Linsenformabschnitt (12) kontaktiert;
das Befestigen des einzelnen männlichen Linsenformabschnitts (12) an dem Greifkopf (68);
das Trennen der Greifkopf- Anordnung (66) und des einzelnen männlichen Linsenformabschnitts (12) von der ersten Stapelplatte (10);
das Bewegen der getrennten Greifkopf- Anordnung (66) und des einzelnen männlichen Linsenformabschnitts (12) in Eingriff mit einer zweiten Stapelplatte (40), welche einen weiblichen Linsenformabschnitt (38), der eine zweite optische Oberfläche (78) aufweist, trägt , wobei die zweite Stapelplatte (40) eine Vielzahl von zweiten Löchern (42) aufweist, die so bemessen und positioniert sind, um die Vielzahl von beabstandeten Ausrichtungsstiften (70) aufzunehmen, wobei das Bewegen bewirkt, dass der einzelne männliche Linsenformabschnitt (12) mit dem weiblichen Linsenformabschnitt (38), welcher von der zweiten Stapelplatte (40) getragen wird, in Ausrichtung kommt, wenn die Vielzahl der beabstandeten Ausrichtungsstifte (70) in der Vielzahl von zweiten Löchern (42) aufgenommen werden und bevor der einzelne männliche Linsenformabschnitt (12) den weiblichen Linsenformabschnitt (38) kontaktiert; und
das Anordnen der ausgerichteten männlichen und weiblichen Linsenformabschnitte (12), (38) in Kontakt miteinander;
wobei die zweite Stapelplatte (40) ferner einen zweiten Hohlraum (44) aufweist, der so bemessen und positioniert ist, um den zweiten Linsenformabschnitt (38) und einen umlaufenden erhöhten Abschnitt (46) in dem Hohlraum (44) aufzunehmen, wobei der erhöhte Abschnitt (46) bewirkt, dass die Ausrichtung des weiblichen Linsenformabschnitts (38) in dem Hohlraum (44) mit dem einzelnen männlichen Linsenformabschnitt (12) unterstützt wird, ohne dass der einzelne männliche Linsenformabschnitt (12) die zweite optische Oberfläche (78) des weiblichen Linsenformabschnitts (38) kontaktiert.

2. Das Verfahren nach Anspruch 1, welches ferner umfasst:
das Bereitstellen einer polymerisierbaren Zusammensetzung zwischen den ausgerichteten männlichen und weiblichen Linsenformabschnitten (12), (38), die eine Anordnung zur Fertigung bilden; und
das Umsetzen der polymerisierbaren Zusammensetzung, um einen polymeren Kontaktlinsenkörper zu bilden.

3. Das Verfahren nach Anspruch 1, wobei der Greifkopf (68) eine Längsachse aufweist und sich die Greifkopf- Anordnung (66) im Wesentlichen frei in einer Ebene senkrecht zu der Längsachse des Greifkopfes (68) bewegt.

4. Das Verfahren nach Anspruch 3, wobei die im Wesentlichen freie Bewegung der Greifkopf- Anordnung (66) in einer Ebene senkrecht zu der Längsachse des Greifkopfes (68) mindestens eines der folgenden vereinfacht: das in Ausrichtung bringen des Greifkopfes (68) mit dem einzelnen männlichen Linsenformabschnitt (12); das in Ausrichtung bringen des einzelnen männlichen Linsenformabschnitts (12) und des weiblichen Linsenformabschnitts (38).

5. Das Verfahren nach Anspruch 1, wobei die erste Stapelplatte (10), die zweite Stapelplatte (40) und die Greifkopf- Anordnung (66) so konfiguriert sind, dass die erste optische Oberfläche des einzelnen männlichen Linsenformabschnitts (12) nicht mit irgendeiner Oberfläche in Kontakt kommt, wenn die erste Stapelplatte (10) den einzelnen männlichen Linsenformabschnitt (12) trägt, wenn der einzelne männliche Linsenformabschnitt (12) an den Greifkopf (68) befestigt wird, während des Schritts des Trennens, während des Schritts des Bewegens und während des abschließenden Schritts.

6. Das Verfahren nach Anspruch 1, wobei die erste Stapelplatte (10) einen ersten Hohlraum (16) aufweist, welcher so bemessen und positioniert ist, um den einzelnen männlichen Linsenformabschnitt (12) und einen umlaufenden Rand (18), welcher in den Hohlraum (16) eingelassen ist, aufzunehmen, wobei der Rand (18) im Wesentlichen zu der Vielzahl von ersten Löchern (20), welche so bemessen und positioniert sind, um die Vielzahl von beabstandeten Ausrichtungsstiften (70) aufzunehmen, benachbart ist, und wobei der Rand (18) bewirkt, dass die Ausrichtung des einzelnen männlichen Linsenformabschnitts (12) in dem Hohlraum (16), unterstützt wird, ohne dass die erste optische Oberfläche des einzelnen männlichen Linsenformabschnitts (12) kontaktiert wird.

7. Das Verfahren nach Anspruch 2, wobei der Schritt des Bereitstellens das Anordnen der polymerisierbaren Zusammensetzung auf dem weiblichen Linsenformabschnitt (38) zu mindestens einem der folgenden Zeitpunkte umfasst: vor dem Schritt des Bewegens, während des Schritts des Bewegens, und nach dem Schritt des Bewegens.

8. Das Verfahren nach Anspruch 1, wobei der weibliche Linsenformabschnitt (38) an der Außenseite der Form eine Randleiste (54) beinhaltet, und wobei der Schritt des Bewegens bewirkt, dass die Vielzahl der beabstandeten Ausrichtungsstifte (70) mit der Randleiste (54) an der Außenseite der Form in Kontakt gebracht wird, bevor die Vielzahl der beabstandeten Ausrichtungsstifte (70) von der Vielzahl der zweiten Löcher (42) aufgenommen werden.

9. Das Verfahren nach Anspruch 1, wobei die erste Stapelplatte (10) eine Vielzahl von männlichen Linsenformabschnitten (12) trägt, und wobei die zweite Stapelplatte (40) mit einer Vielzahl von weiblichen Linsenformabschnitten (38) versehen ist.

10. Das Verfahren nach Anspruch 1, wobei der Schritt des Bewegens bewirkt, dass der einzelne männliche Linsenformabschnitt (12) in Ausrichtung mit dem weiblichen Linsenformabschnitt (38) kommt, ohne dass die erste optische Oberfläche die zweite optische Oberfläche (78) kontaktiert.

11. Das Verfahren nach Anspruch 1, wobei die erste Stapelplatte (10) mindestens ein erstes strukturelles Merkmal beinhaltet, das es dem Greifkopf (68) erleichtert, in Ausrichtung mit dem einzelnen männlichen Linsenformabschnitt (12) zu kommen, und / oder die zweite Stapelplatte (40) mindestens ein zweites strukturelles Merkmal beinhaltet, das es dem einzelnen männlichen Linsenformabschnitt (12) erleichtert, in Ausrichtung mit dem weiblichen Linsenformabschnitt (38) zu kommen.

12. Das Verfahren nach Anspruch 1, wobei der einzelne männliche Linsenformabschnitt (12) an einem ersten länglichen Flusskanal (14) angebracht ist, und wobei die erste Stapelplatte (10) beinhaltet: eine Vielzahl von beabstandeten ersten erhöhten Segmenten (26), welche einen definierten Weg für die Anordnung des ersten länglichen Flusskanals (14) bilden, um das Platzieren des einzelnen männlichen Linsenformabschnitts (12) auf der ersten Stapelplatte (10) zu vereinfachen.

13. Das Verfahren nach Anspruch 12, wobei die erste Stapelplatte (10) eine Öffnung umfasst, um es dem ersten länglichen Flusskanal (14) zu ermöglichen, die erste Stapelplatte (10) zu durchlaufen, wenn der längliche Flusskanal (14) von dem einzelnen männlichen Linsenformabschnitt (12) losgelöst ist.

14. Das Verfahren nach Anspruch 1, wobei die Vielzahl von beabstandeten Ausrichtungsstiften (70) um den Greifkopf (68) herum positioniert ist.

15. System zur Herstellung einer Kontaktlinse, wobei das System umfasst:
eine Anordnung mit Greifkopf (66), die einen einzelnen Greifkopf (68) und eine Vielzahl von beabstandeten Ausrichtungsstiften (70) aufweist, wobei die Greifkopf-Anordnung (66) so strukturiert ist, dass sie an einen einzelnen männlichen Linsenformabschnitt (12) befestigt werden kann;
eine erste Stapelplatte (10), welche eine Vielzahl von männlichen Kontaktlinsenformabschnitten (12) trägt und eine Vielzahl von ersten Löchern (20), die so bemessen und positioniert sind, um die Vielzahl der beabstandeten Ausrichtungsstifte (70) aufzunehmen, aufweist;
eine Vakuumquelle (72), die mit dem Greifkopf (68) funktionsfähig verbunden ist und betrieben werden kann, um einen einzelnen männlichen Linsenformabschnitt (12) an dem Greifkopf (68) zu befestigen; und
eine zweite Stapelplatte (40), welche einen weiblichen Linsenformabschnitt (38) trägt und eine Vielzahl von zweiten Löchern (42), die so bemessen und positioniert sind, um die Vielzahl von beabstandeten Ausrichtungsstiften (70) aufzunehmen, aufweist, wobei die zweite Stapelplatte (40) einen zweiten Hohlraum (44) aufweist, der so bemessen und positioniert ist, um den weiblichen Linsenformabschnitt (38) aufzunehmen, **dadurch gekennzeichnet, dass**
die Greifkopf- Anordnung (66) einen einzelnen Greifkopf (68) aufweist, und die zweite Stapelplatte (40) einen umlaufenden erhöhten Abschnitt (46) innerhalb des zweiten Hohlraums (44) aufweist, wobei der erhöhte Abschnitt (46) bewirkt, dass die Ausrichtung des weiblichen Linsenformabschnitts (38) in dem zweiten Hohlraum (44) mit dem männlichen Linsenformabschnitt (12), der an der Greifkopf-Anordnung (66) befestigt ist, unterstützt wird, ohne dass der einzelne männliche Linsenformabschnitt (12) eine zweite optische Oberfläche (78) des weiblichen Linsenformabschnitts (38) kontaktiert, wobei die Greifkopf- Anordnung (66) und der befestigte einzelne männliche Linsenformabschnitt (12) in Eingriff mit der zweiten Stapelplatte (40) bewegt werden können, so dass der einzelne männliche Linsenformabschnitt (12) in Ausrichtung mit dem weiblichen Linsenformabschnitt (38) kommt, wenn die Vielzahl der beabstandeten Ausrichtungsstifte (70) in der Vielzahl von zweiten Löchern (42) aufgenommen werden.

16. Das System nach Anspruch 15, wobei der Greifkopf (68) eine Längsachse aufweist und die Greifkopf- Anordnung (66) im Wesentlichen frei in einer Ebene senkrecht zu der Längsachse des Greifkopfes (68) beweglich ist.

17. Das System nach Anspruch 15, wobei die erste Stapelplatte (10) einen ersten Hohlraum (16), der so bemessen und positioniert ist, um den männlichen Linsenformabschnitt (12) und einen umlaufenden Rand (18), der in den Hohlraum (16) eingelassen ist, aufzunehmen, wobei der Rand (18) im Wesentlichen zu der Vielzahl von ersten Löchern (20), die so bemessen und positioniert sind, um die Vielzahl von beabstandeten Ausrichtungsstiften (70) aufzunehmen, benachbart ist, und wobei der Rand (18) bewirkt, dass die Ausrichtung des männlichen Linsenformabschnitts (12) in dem Hohlraum (16) unterstützt wird, ohne dass die erste optische Oberfläche des männlichen Linsenformabschnitts (12) kontaktiert wird.

18. Das System nach Anspruch 15, wobei die Vielzahl von beabstandeten Ausrichtungsstiften (70) um den Greifkopf (68) herum positioniert ist.

## Revendications

1. Procédé de fabrication d'une lentille de contact, le procédé étant **caractérisé par** les étapes combinées de :
placement d'un ensemble de tête de prélèvement (66) ayant une tête de prélèvement unique (68) et une pluralité de broches d'alignement espacées (70) en engagement avec une première palette (10) portant une pluralité de sections de moules mâles pour lentilles de contact (12), chaque section de moule mâle pour lentilles de contact (12) ayant une première surface optique, la première palette (10) ayant une pluralité de premiers trous (20) dimensionnés et positionnés pour recevoir la pluralité de broches d'alignement espacées (70), le placement étant efficace, de sorte que la tête de prélèvement (68) vient en alignement avec une section de moule mâle pour lentilles de contact, unique (12) supportée par la première palette (10) lorsque la pluralité de broches d'alignement espacées (70) est reçue par la pluralité de premiers trous (20) et avant que la tête de prélèvement (68) n'entre en contact avec la section de moule mâle pour lentilles de contact (12) ;
fixation de la section de moule mâle pour lentilles de contact, unique (12) à la tête de prélèvement (68) ;
séparation de l'ensemble de tête de prélèvement (66) et de la section de moule mâle pour lentilles de contact, unique (12) de la première palette (10) ;
déplacement de l'ensemble de tête de prélèvement (66) et de la section de moule mâle pour lentilles de contact, unique (12) en engagement avec une seconde palette (40) portant une section de moule femelle pour lentilles de contact (38) ayant une seconde surface optique (78), la seconde palette (40) ayant une pluralité de seconds trous (42) dimensionnés et positionnés pour recevoir la pluralité de broches d'alignement espacées (70), le déplacement étant efficace, de sorte que la section de moule mâle pour lentilles de contact, unique (12), vient en alignement avec une section de moule femelle pour lentilles (38) supportée par la seconde palette (40) lorsque la pluralité de broches d'alignement espacées (70) est reçue dans la pluralité de seconds trous (42) et avant que la section de moule mâle pour lentilles de contact, unique (12) n'entre en contact avec la section de moule femelle pour lentilles (38) ; et
placement des sections de moules pour lentilles mâle et femelle alignées (12), (38) en contact l'une avec l'autre ;
dans lequel la seconde palette (40) a également une seconde cavité (44) dimensionnée et positionnée pour recevoir la section de moule femelle pour lentilles (38), et une partie surélevée circonférentielle (46) à l'intérieur de la cavité (44), la partie surélevée (46) étant efficace pour aider à aligner la section de moule femelle pour lentilles (38) dans la cavité (44) avec la section de moule mâle pour lentilles de contact, unique (12), sans que la section de moule mâle pour lentilles de contact, unique (12), n'entre en contact avec la seconde surface optique (78) de la section de moule femelle pour lentilles (38).

2. Procédé de la revendication 1, qui comprend, en outre :
la fourniture d'une composition polymérisable entre les sections de moules mâle et femelle pour lentilles alignées (12), (38), formant un ensemble de moule ; et
la réaction de la composition polymérisable pour former un corps de lentille de contact polymère.

3. Procédé de la revendication 1, dans lequel la tête de prélèvement (68) a un axe longitudinal et dans lequel l'ensemble de tête de prélèvement (66) se déplace sensiblement librement dans un plan perpendiculaire à l'axe longitudinal de la tête de prélèvement (68).

4. Procédé de la revendication 3, dans lequel le déplacement de l'ensemble de tête de prélèvement (66) sensiblement librement dans un plan perpendiculaire à l'axe longitudinal de la tête de prélèvement (68) facilite au moins un de la mise en alignement de la tête de prélèvement (68) avec la section de moule mâle pour lentilles, unique (12) et la mise en alignement de la section de moule mâle pour lentilles, unique (12) et la section de moule femelle pour lentilles (38).

5. Procédé de la revendication 1, dans lequel la première palette (10), la seconde palette (40) et l'ensemble de tête de prélèvement (66) sont configurés de sorte que la première surface optique de la section de moule mâle pour lentilles, unique (12) ne vient en contact avec aucune surface lorsque la première palette (10) supporte la section de moule mâle pour lentilles, unique (12), lorsque la section de moule mâle pour lentilles, unique (12) est fixée à la tête de prélèvement (68), pendant l'étape de séparation, pendant l'étape de déplacement et pendant l'étape de fermeture.

6. Procédé de la revendication 1, dans lequel la première palette (10) a une première cavité (16) dimensionnée et positionnée pour recevoir la section de moule mâle pour lentilles, unique (12), et un bord périphérique (18) ménagé à l'intérieur de la cavité (16), le bord (18) étant sensiblement adjacent à la pluralité de premiers trous (20) dimensionnés et positionnés pour recevoir la pluralité de broches d'alignement espacées (70), et le bord (18) étant efficace pour aider à aligner la section de moule mâle pour lentilles, unique (12) dans la cavité (16) sans entrer en contact avec la première surface optique de la section de moule mâle pour lentilles, unique (12).

7. Procédé de la revendication 2, dans lequel l'étape de fourniture comprend le placement de la composition polymérisable sur la section de moule femelle pour lentilles (38) à au moins un des moments suivants : avant l'étape de déplacement, pendant l'étape de déplacement, et après l'étape de déplacement.

8. Procédé de la revendication 1, dans lequel la section de moule femelle pour lentilles (38) inclut une jupe extérieure de moule (54), et l'étape de déplacement est efficace pour faire entrer en contact la pluralité de broches espacées (70) avec la jupe extérieure de moule (54) avant que la pluralité de broches espacées (70) ne soit reçue par la pluralité de second trous (42).

9. Procédé de la revendication 1, dans lequel la première palette (10) porte une pluralité de sections de moules mâles pour lentilles (12), et dans lequel la seconde palette (40) maintient une pluralité de sections de moules femelles pour lentilles (38).

10. Procédé de la revendication 1, dans lequel l'étape de déplacement est efficace de sorte que la section de moule mâle pour lentilles, unique (12) vient en alignement avec la section de moule femelle pour lentilles (38) sans que la première surface optique ne vienne en contact avec la seconde surface optique (78).

11. Procédé de la revendication 1, dans lequel la première palette (10) inclut au moins une première caractéristique structurelle pour faciliter la mise en alignement de la tête de prélèvement (68) avec la section de moule mâle pour lentilles, unique (12), et / ou la seconde palette (40) inclut au moins une seconde caractéristique structurelle pour faciliter la mise en alignement de la section de moule mâle pour lentilles, unique (12), avec la section de moule femelle pour lentilles (38).

12. Procédé de la revendication 1, dans lequel la section de moule mâle pour lentilles, unique (12) est fixée à une première glissière allongée (14) et la première palette (10) inclut une pluralité de premiers segments surélevés espacés (26) formant un passage défini pour placer la première glissière allongée (14) afin de faciliter le placement de la section de moule mâle pour lentilles, unique (12) sur la première palette (10).

13. Procédé de la revendication 12, dans lequel la première palette (10) comprend une ouverture pour permettre à la première glissière allongée (14) de passer à travers la première palette (10) lorsque la glissière allongée (14) est détachée de la section de moule mâle pour lentilles, unique (12).

14. Procédé de la revendication 1, dans lequel la pluralité de broches d'alignement espacées (70) est positionnée autour de la tête de prélèvement (68).

15. Système de fabrication d'une lentille de contact, le système comprenant :
un ensemble de tête de prélèvement (66) ayant une tête de prélèvement simple (68) et une pluralité de broches d'alignement espacées (70), l'ensemble de tête de prélèvement (66) étant structuré pour être fixé à une section de moule mâle pour lentilles, unique (12) ;
une première palette (10) portant une pluralité de sections de moules mâles pour lentilles de contact (12) et ayant une pluralité de premiers trous (20) dimensionnés et positionnés pour recevoir la pluralité de broches d'alignement espacées (70) ;
une source de vide (72) couplée en fonctionnement à la tête de prélèvement (68) et pouvant être actionnée pour fixer une section de moule mâle pour lentilles, unique (12) à la tête de prélèvement (68) ; et
une seconde palette (40) portant une section de moule femelle pour lentilles (38) et ayant une pluralité de seconds trous (42) dimensionnés et positionnés pour recevoir la pluralité de broches d'alignement espacées (70), la seconde palette (40) ayant une seconde cavité (44) dimensionnée et positionnée pour recevoir la section de moule femelle pour lentilles (38), **caractérisée par le fait que**
l'ensemble de tête de prélèvement (66) a une tête de prélèvement unique (68), et que la seconde palette (40) a une partie surélevée circonférentielle (46) à l'intérieur de la seconde cavité (44), la partie surélevée (46) étant efficace pour aider à aligner la section de moule femelle pour lentilles (38) dans la seconde cavité (44) avec la section de moule mâle pour lentilles de contact, unique (12), fixée à l'ensemble de tête de prélèvement (66) sans que la section de moule mâle pour lentilles de contact, unique (12) n'entre en contact avec une
seconde surface optique (78) de la section de moule femelle pour lentilles (38), dans laquelle l'ensemble de tête de prélèvement (66) et la section de moule mâle pour lentilles, unique fixée (12), peuvent être déplacés en engagement avec la seconde palette (40), de sorte que la section de moule mâle pour lentilles, unique (12) vient en alignement avec la section de moule femelle pour lentilles (38) lorsque la pluralité de broches d'alignement espacées (70) est reçue dans la pluralité de seconds trous (42).

16. Système de la revendication 15, dans lequel la tête de prélèvement (68) a un axe longitudinal et l'ensemble de tête de prélèvement (66) se déplace sensiblement librement dans un plan perpendiculaire à l'axe longitudinal de la tête de prélèvement (68).

17. Système de la revendication 15, dans lequel la première palette (10) a une première cavité (16) dimensionnée et positionnée pour recevoir la section de moule mâle pour lentilles (12), et un bord périphérique (18) ménagé à l'intérieur de la cavité (16), le bord (18) étant sensiblement adjacent à la pluralité de premiers trous (20) dimensionnés et positionnés pour recevoir la pluralité de broches d'alignement espacées (70), et le bord (18) étant efficace pour aider à aligner la section de moule mâle pour lentilles (12) dans la cavité (16) sans entrer en contact avec la première surface optique de la section de moule mâle pour lentilles (12).

18. Système de la revendication 15, dans lequel la pluralité de broches d'alignement espacées (70) est positionnée autour de la tête de prélèvement (68).
